# EUROPEAN PATENT APPLICATION

(11) **EP 3 906 997 A2**
(43) Date of publication of application: **10.11.2021**
(21) Application number: 21172425.7
(22) Date of filing: 06.05.2021
(51) Int. Cl.: B01J 20/28, B01J 20/286, B01J 20/32, C07H 21/00

(54) **MACROPOROUS CONTROLLED POROSITY SILICA GEL SUITABLE FOR OLIGONUCLEOTIDE SYNTHESIS**

(30) Priority: 07.05.2020 US 202063021376 P
(71) Applicant: Chemgenes Corporation, Wilmington, MA 01887 (US)
(72) Inventor: LAIKHTER, ANDREI, Wilmington, Massachusetts, 01887 (US); SRIVASTAVA, SURESH C., Wilmington, Massachusetts, 01887 (US)
(74) Representative: J A Kemp LLP

(57) **Abstract**

Disclosed herein is macroporous controlled porosity silica gel (CPSG) covalently modified with a variety of moieties (e.g., moieties suitable for oligonucleotide synthesis comprising a spacer, a linker and a nucleoside, chromophore, ligand or bioconjugation linker, or a spacer and a universal linker). Also disclosed herein are methods of making the covalently-modified, macroporous CPSG, and methods of using the covalently-modified, macroporous CPSG to synthesize oligonucleotides.

SEQUENCE LISTING

<110>  CHEMGENES CORPORATION

<120>  MACROPOROUS CONTROLLED POROSITY SILICA GEL SUITABLE FOR
       OLIGONUCLEOTIDE SYNTHESIS

<130>  N421764EP

<150>  US 63/021,376
<151>  2020-05-07

<160>  3

<170>  PatentIn version 3.5

<210>  1
<211>  21
<212>  DNA
<213>  Artificial Sequence

<220>
<223>  Oligonucleotide Sequence

<400>  1
tcagtgcacg atagtcgcat t                                                21


<210>  2
<211>  100
<212>  DNA
<213>  Artificial Sequence

<220>
<223>  Oligonucleotide Sequence

<400>  2
cactccacta caactacatg tgtaacagtt cctccatatg ttcctcctcc atccactact      60

tgccatccac tacaactaca tgtgtaacag ttcctccata                            100


<210>  3
<211>  12
<212>  RNA
<213>  Artificial Sequence

<220>
<223>  Oligonucleotide Sequence

<400>  3
gaguucauag cu                                                          12

## Description

### RELATED APPLICATION

This application claims the benefit of U.S. Provisional Application No. 63/021,376, filed on May 7, 2020. The entire teachings of this application are incorporated herein by reference.

### INCORPORATION BY REFERENCE OF MATERIAL IN ASCII TEXT FILE

This application incorporates by reference the Sequence Listing contained in the following ASCII text file being submitted concurrently herewith:
a) File name: N421764EP CJM EGH_ST25.txt, created 4 May 2021, 1 KB in size.

### BACKGROUND

Controlled porosity glass (CPG) is a macroporous, silica-based solid support commonly used in oligonucleotide synthesis. However, CPG is very fragile and difficult to use, and leaches small glass particles into liquid chromatography pumps used in large-scale oligonucleotide synthesis. Even gentle shaking tends to generate smaller glass particles, and therefore sacrifices the quality of the specific optimal target size.

Macroporous polystyrene is another solid support used in oligonucleotide synthesis. Macroporous polystyrene swells significantly in organic solvents during oligonucleotide synthesis, and requires adjustable bed size columns in the case of medium- and large-scale synthesizers. It also shrinks in aqueous media during cleavage and base deprotection, which reduces recovery of the full-length product.

Microporous silica gel (*e.g.,* Waters PORASIL® B and C, pore size approximately 100 Å) has also been used in oligonucleotide synthesis. However, the longest oligonucleotide was synthesized by phosphochloridite chemistry and was only fifteen nucleobases long.

Accordingly, there is a need for other solid supports for oligonucleotide synthesis that overcome one or more of the aforementioned disadvantages.

### SUMMARY

The present disclosure relates to covalently-modified, macroporous controlled porosity silica gel (CPSG), and methods for making and/or using the same, *e.g.,* for oligonucleotide synthesis.

One embodiment provides macroporous CPSG covalently modified with a moiety represented by one of the following structural formulas: 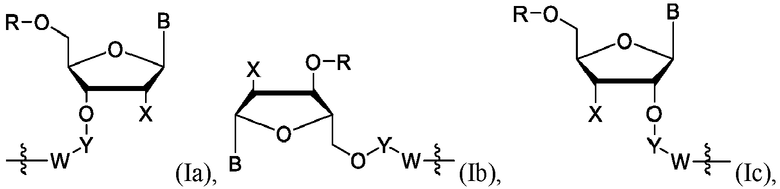 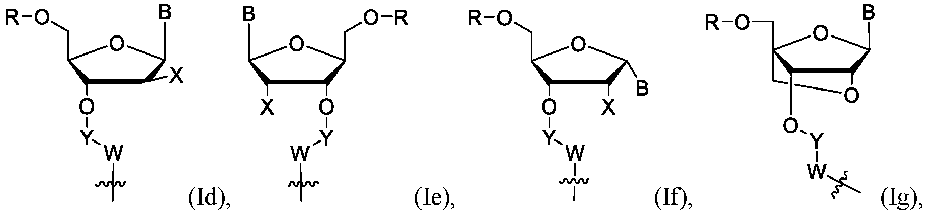 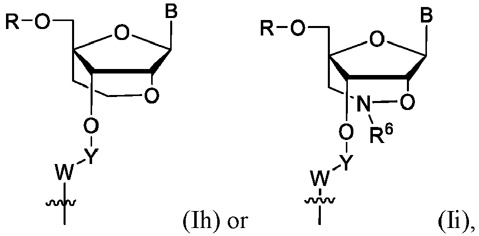 wherein values for the variables (*e.g*., B, R, R⁶, W, X, Y) are as described herein.

Another embodiment provides macroporous CPSG covalently modified with a moiety represented by the following structural formula: 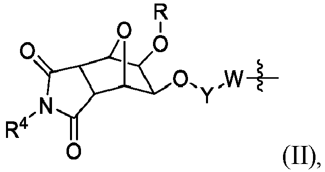 wherein values for the variables (*e.g*., R, R⁴, W, Y) are as described herein.

Yet another embodiment provides macroporous CPSG covalently modified with a moiety represented by the following structural formula: 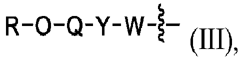 wherein values for the variables (*e.g*., Q, R, W, Y) are as described herein.

Also provided is a method of synthesizing an oligonucleotide. The method comprises providing covalently-modified, macroporous CPSG disclosed herein, attaching one or more nucleotides to the macroporous CPSG, thereby synthesizing an oligonucleotide covalently attached to the macroporous CPSG, and cleaving the oligonucleotide covalently attached to the macroporous CPSG from the macroporous CPSG, thereby synthesizing the oligonucleotide.

Also provided is a method of making covalently-modified, macroporous CPSG disclosed herein. The method comprises modifying one or more hydroxyl groups of macroporous CPSG with a spacer selected from (R²)₃Si-(C₁-C₂₅)alkylene-Z¹, (R²)₃Si-(C₂-C₂₅)alkenylene-Z¹, (R²)₃Si-(C₂-C₂₅)alkynylene-Z¹, (R²)₃Si-(C₁-C₂₅)heteroalkylene-Z¹, (R²)₃Si-(C₂-C₂₅)heteroalkenylene-Z¹ or (R²)₃Si-(C₂-C₂₅)heteroalkynylene-Z¹, wherein each R² is independently -O(C₁-C₁₀)alkyl or -O(C₆-C₁₂)aryl; Z¹ is NH₂, OP¹ or SP²; P¹ is a hydroxyl protecting group; and P² is a sulfhydryl protecting group, to obtain macroporous CPSG covalently modified with a spacer selected from 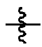 (R³)₂Si-(C₁-C₂₅)alkylene-Z¹, 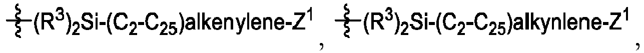 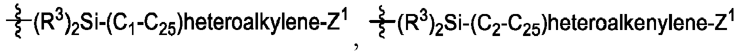 or 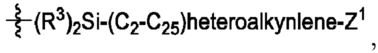 respectively, wherein 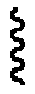 indicates the point of attachment of the spacer to the macroporous CPSG; each R³ is independently an additional point of attachment of the spacer to the macroporous CPSG, or R²; and R² and Z¹ are as described above. Unsilylated hydroxyl groups of the macroporous CPSG covalently modified with the spacer are capped with a silanizing agent, and P¹ and P², if present, are removed with a deprotecting agent to obtain capped and functionalized macroporous CPSG. A compound of one of the following structural formulas: 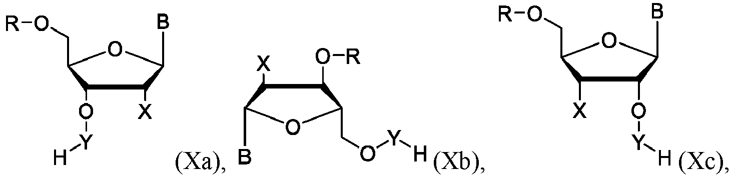 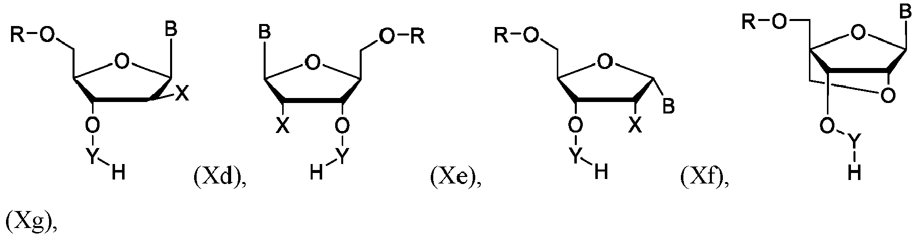 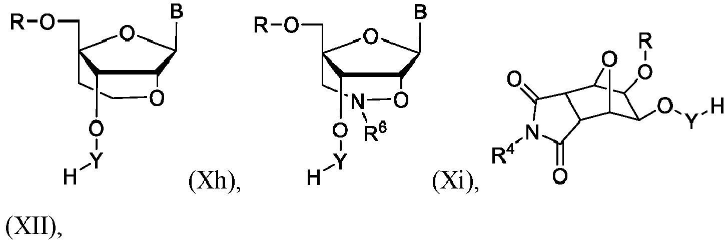 (XI) or R-O-Q-Y-H wherein R is 4,4'-dimethoxytrityl and R⁴, R⁶, B, Q, X and Y are as described herein, is reacted with the capped and functionalized macroporous CPSG in the presence of a coupling reagent in an organic solvent, thereby making the covalently-modified, macroporous CPSG.

The covalently-modified, macroporous CPSG disclosed herein significantly improved oligonucleotide syntheses based on phosphoramidite chemistry conducted at various scales, including small *(e.g.,* about 1 micromole) and medium *(e.g.,* greater than 200 micromoles) scales, using various synthesizers. For example, synthesis efficiencies of greater than 99% per step, and n-1 and n+1 closed impurities of less than 1% in crude material were achieved with the covalently-modified, macroporous CPSG disclosed herein.

### DETAILED DESCRIPTION

A description of example embodiments follows.

### Definitions

As used herein, singular articles such as "a," "an" and "the," and similar referents are to be construed to cover both the singular and the plural, unless otherwise indicated herein or clearly contradicted by context. For example, reference to "a moiety" may refer to one or more moieties. When a referent refers to the plural, the members of the plural can be the same as or different from one another. Thus, for example, each of more than one "moiety" can be the same as one another, or each of more than one moiety can be independently selected, such that the moieties are different from one another.

"About" means within an acceptable error range for the particular value, as determined by one of ordinary skill in the art. Typically, an acceptable error range for a particular value depends, at least in part, on how the value is measured or determined, e.g., the limitations of the measurement system. For example, "about" can mean within an acceptable standard deviation, per the practice in the art. Alternatively, "about" can mean a range of ± 20%, ± 10%, ± 5% or ± 1% of a given value. It is to be understood that the term "about" can precede any particular value specified herein, except for particular values used in the Exemplification. Thus, in some embodiments, a mean pore size of 1,000 Å is a mean pore size of about 1,000 Å.

"Acetyl" refers to -C(O)CH₃.

"Alkyl" refers to a saturated, aliphatic, branched or straight-chain, monovalent, hydrocarbon radical having the indicated number of carbon atoms, for example, from 1 to 100 carbon atoms, from 1 to 50 carbon atoms, from 1 to 25 carbon atoms, from 1 to 10 carbon atoms or, in some embodiments, from 1 to 5 carbon atoms. Thus, "(C₁-C₂₅)alkyl" means a radical having from 1-25 carbon atoms in a linear or branched arrangement. Examples of straight chain alkyl groups include, but are not limited to, methyl, ethyl, n-propyl, n-butyl, n-pentyl, n-hexyl, n-heptyl, and n-octyl. Examples of branched alkyl groups include, but are not limited to, isopropyl, iso-butyl, sec-butyl, tert-butyl, neopentyl, isopentyl, and 2,2-dimethylpropyl groups.

"Alkylene" refers to a saturated, aliphatic, branched or straight-chain, divalent, hydrocarbon radical having the indicated number of carbon atoms, for example, from 1 to 100 carbon atoms, from 1 to 50 carbon atoms, from 1 to 25 carbon atoms, from 1 to 10 carbon atoms or, in some embodiments, from 1 to 5 carbon atoms. Thus, "(C₁-C₂₅)alkylene" means a diradical having from 1-25 carbon atoms in a linear or branched arrangement. Examples of alkylene include, but are not limited to, methylene, ethylene (*e.g*., 1,2-ethylene, 1,1-ethylene), propylene, butylene, pentylene, and the like.

"Alkenyl" refers to an aliphatic, branched or straight-chain, monovalent, hydrocarbon radical having at least one carbon-carbon double bond and the indicated number of carbon atoms, for example, from 2 to 100 carbon atoms, from 2 to 50 carbon atoms, from 2 to 25 carbon atoms, from 2 to 10 carbon atoms or, in some embodiments, from 2 to 5 carbon atoms. Thus, "(C₂-C₂₅)alkenyl" means a radical having at least one carbon-carbon double bond and from 2 to 25 carbon atoms in a linear or branched arrangement. In some embodiments, the alkenyl group has one, two, or three carbon-carbon double bonds. Examples include, but are not limited to, vinyl, allyl, -CH=CH(CH₃), -CH=C(CH₃)₂, -C(CH₃)=CH₂, -C(CH₃)=CH(CH₃), -C(CH₂CH₃)=CH₂, among others.

"Alkenylene" refers to an aliphatic, branched or straight-chain, divalent, hydrocarbon radical having at least one carbon-carbon double bond and the indicated number of carbon atoms, for example, from 2 to 100 carbon atoms, from 2 to 50 carbon atoms, from 2 to 25 carbon atoms, from 2 to 10 carbon atoms or, in some embodiments, from 2 to 5 carbon atoms. Thus, "(C₂-C₂₅)alkenylene" means a diradical having at least one carbon-carbon double bond and from 2 to 25 carbon atoms in a linear or branched arrangement. In some embodiments, the alkenylene group has one, two, or three carbon-carbon double bonds. Alkenylene includes, but is not limited to, ethenylene and isoprenylene.

"Alkynyl" refers to an aliphatic, branched or straight-chain, monovalent, hydrocarbon radical having at least one carbon-carbon triple bond and the indicated number of carbon atoms, for example, from 2 to 100 carbon atoms, from 2 to 50 carbon atoms, from 2 to 25 carbon atoms, from 2 to 10 carbon atoms or, in some embodiments, from 2 to 5 carbon atoms. Thus, "(C₂-C₂₅)alkynyl" means a radical having at least one carbon-carbon triple bond and from 2 to 25 carbon atoms in a linear or branched arrangement. In some embodiments, the alkynyl group has one, two, or three carbon-carbon triple bonds. Examples include, but are not limited to, -C≡CH, -C≡CCH₃, -CH₂C≡CCH₃, -C≡CCH₂CH(CH₂CH₃)₂, among others.

"Alkynylene" refers to an aliphatic, branched or straight-chain, divalent, hydrocarbon radical having at least one carbon-carbon triple bond and the indicated number of carbon atoms. Thus, "(C₂-C₂₅)alkynylene" means a diradical having at least one carbon-carbon triple bond and from 2 to 25 carbon atoms in a linear or branched arrangement. In some embodiments, the alkynylene group has one, two, or three carbon-carbon triple bonds. Alkynylene includes, but is not limited to, propargylene.

"Alkoxy" refers to the -O-alkyl radical, wherein alkyl is as defined herein.

"Amino" refers to -NH₂.

"Amino protecting group" refers to a chemical group(s) that replaces at least one hydrogen of an amino group and decreases the reactivity of the nitrogen atom of the amino group under at least certain synthetic conditions. Examples of amino protecting groups can be found, for example, in Wuts, P.G.M. Protecting Groups in Organic Synthesis, 5th Ed., New York, John Wiley & Sons, 2014. Mono-protected aminos are aminos in which one hydrogen atom of the amino group is replaced with the chemical group that decreases the reactivity of the nitrogen atom of the amino group under at least certain synthetic conditions. Examples of mono-protected aminos include acylamino *(e.g.,* acetylamino), amidines *(e.g., N,N*-dimethylformamidine, *N,N*-dibutylformamidine), t-butyloxycarbonylamino (-N(H)BOC), ethyloxycarbonylamino, methyloxycarbonylamino, trichloroethyloxycarbonylamino, allyloxycarbonylamino (-N(H)Alloc), benzyloxocarbonylamino (-N(H)CBZ), allylamino, benzylamino (-N(H)Bn), fluorenylmethylcarbonyl (-N(H)Fmoc), formamido, acetamido, chloroacetamido, dichloroacetamido, trichloroacetamido, phenylacetamido, trifluoroacetamido, benzamido, t-butyldiphenylsilyl, and the like. Di-protected aminos are aminos in which two hydrogen atoms of the amino group are replaced with the chemical group(s) that decreases the reactivity of the nitrogen atom of the amino group under at least certain synthetic conditions, and include aminos independently protected with the mono-protecting groups described above that produce mono-protected aminos, and further include cyclic imides, such as phthalimide, maleimide, succinimide, and the like.

"Aryl" refers to a monocyclic, bicyclic or tricyclic, carbocyclic, monovalent aromatic ring radical having the indicated number of ring atoms, for example, from six to 15 or from six to 12 ring atoms. Thus, "(C₆-C₁₂)aryl" means a ring radical having from 6-12 ring atoms. Aryl groups include, but are not limited to, phenyl, azulenyl, heptalenyl, biphenyl, fluorenyl, phenanthrenyl, anthracenyl, indenyl, indanyl, pentalenyl, and naphthyl. In some embodiments, aryl is phenyl or naphthyl (*e.g*., phenyl).

"Deprotecting agent" refers to a chemical agent suitable to remove a protecting group from a functional group. Deprotecting agents are well-known in the art and can be found, for example, in Wuts, P.G.M. Protecting Groups in Organic Synthesis, 5th Ed., New York, John Wiley & Sons, 2014. For example, various acids are useful for removing acid-labile protecting groups from a hydroxyl or thiol functional group. Fluoride ion is often useful for removing a silyl protecting group from a functional group, such a hydroxyl or thiol.

"Halogen" and "halo" refer to fluorine, chlorine, bromine and iodine.

"Haloalkyl" refers to alkyl radical wherein one or more hydrogen atoms is replaced with a halogen atom, and alkyl is as described herein. "Haloalkyl" includes mono, poly, and perhaloalkyl groups, wherein each halogen is independently selected from fluorine, chlorine, bromine and iodine (*e.g*., fluorine, chlorine and bromine). In one aspect, haloalkyl is perhaloalkyl (*e.g*., perfluoroalkyl). Haloalkyl includes, but is not limited to, trifluoromethyl and pentafluoroethyl.

"Haloalkoxy" refers to a haloalkyl radical attached through an oxygen linking atom, wherein haloalkyl is as described herein. Haloalkoxy includes, but is not limited to, trifluoromethoxy.

"Heteroalkyl" refers to a saturated, branched or straight-chain, monovalent, hydrocarbon radical having the indicated number of carbon atoms, for example, from 1 to 100 carbon atoms, from 1 to 50 carbon atoms, from 1 to 25 carbon atoms, from 1 to 10 carbon atoms or, in some embodiments, from 1 to 5 carbon atoms, wherein one or more (*e.g*., 1, 2, 3, 4, 5 or 6) of the carbon atoms in the chain is replaced with a heteroatom selected from N, O, S and Si. Thus, "(C₁-C₂₅)heteroalkyl" means a radical having from 1-25 carbon atoms in a linear or branched arrangement wherein one or more (*e.g*., 1, 2, 3, 4, 5 or 6) of the carbon atoms in the chain is replaced with a heteroatom selected from N, O, S and Si. Examples of heteroalkyl groups include, but are not limited to, aminopropyl, aminocaproyl and 1-(((6-aminohexyl)carbamoyl)oxy)hexan-2-yl acetate.

"Heteroalkylene" refers to a saturated, aliphatic, branched or straight-chain, divalent, hydrocarbon radical having the indicated number of carbon atoms, for example, from 1 to 100 carbon atoms, from 1 to 50 carbon atoms, from 1 to 25 carbon atoms, from 1 to 10 carbon atoms or, in some embodiments, from 1 to 5 carbon atoms, wherein one or more (*e.g*., 1, 2, 3, 4, 5 or 6) of the carbon atoms in the chain is replaced with a heteroatom selected from N, O, S and Si. Thus, "(C₁-C₂₅)heteroalkylene" means a diradical having from 1-25 carbon atoms in a linear or branched arrangement, wherein one or more (*e.g*., 1, 2, 3, 4, 5 or 6) of the carbon atoms in the chain is replaced with a heteroatom selected from N, O, S and Si. Examples of heteroalkylene include, but are not limited to, aminopropylene, aminocaproylene and 1-(((6-aminohexyl)carbamoyl)oxy)hexan-2-ylene acetate.

"Heteroalkenyl" refers to an aliphatic, branched or straight-chain, monovalent, hydrocarbon radical having at least one carbon-carbon double bond and the indicated number of carbon atoms, for example, from 2 to 100 carbon atoms, from 2 to 50 carbon atoms, from 2 to 25 carbon atoms, from 2 to 10 carbon atoms or, in some embodiments, from 2 to 5 carbon atoms, wherein one or more (*e.g*., 1, 2, 3, 4, 5 or 6) of the carbon atoms in the chain is replaced with a heteroatom selected from N, O, S and Si. Thus, "(C₂-C₂₅)heteroalkenyl" means a radical having at least one carbon-carbon double bond and from 2 to 25 carbon atoms in a linear or branched arrangement, wherein one or more (*e.g*., 1, 2, 3, 4, 5 or 6) of the carbon atoms in the chain is replaced with a heteroatom selected from N, O, S and Si. In some embodiments, the heteroalkenyl group has one, two, or three carbon-carbon double bonds.

"Heteroalkenylene" refers to an aliphatic, branched or straight-chain, divalent, hydrocarbon radical having at least one carbon-carbon double bond and the indicated number of carbon atoms, for example, from 2 to 100 carbon atoms, from 2 to 50 carbon atoms, from 2 to 25 carbon atoms, from 2 to 10 carbon atoms or, in some embodiments, from 2 to 5 carbon atoms, wherein one or more (*e.g*., 1, 2, 3, 4, 5 or 6) of the carbon atoms in the chain is replaced with a heteroatom selected from N, O, S and Si. Thus, "(C₂-C₂₅)heteroalkenylene" means a diradical having at least one carbon-carbon double bond and from 2 to 25 carbon atoms in a linear or branched arrangement, wherein one or more *(e.g.,* 1, 2, 3, 4, 5 or 6) of the carbon atoms in the chain is replaced with a heteroatom selected from N, O, S and Si. In some embodiments, the heteroalkenylene group has one, two, or three carbon-carbon double bonds.

"Heteroalkynyl" refers to an aliphatic, branched or straight-chain, monovalent, hydrocarbon radical having at least one carbon-carbon triple bond and the indicated number of carbon atoms, for example, from 2 to 100 carbon atoms, from 2 to 50 carbon atoms, from 2 to 25 carbon atoms, from 2 to 10 carbon atoms or, in some embodiments, from 2 to 5 carbon atoms, wherein one or more (*e.g*., 1, 2, 3, 4, 5 or 6) of the carbon atoms in the chain is replaced with a heteroatom selected from N, O, S and Si. Thus, "(C₂-C₂₅)heteroalkynyl" means a radical having at least one carbon-carbon triple bond and from 2 to 25 carbon atoms in a linear or branched arrangement, wherein one or more (*e.g*., 1, 2, 3, 4, 5 or 6) of the carbon atoms in the chain is replaced with a heteroatom selected from N, O, S and Si. In some embodiments, the alkynyl group has one, two, or three carbon-carbon triple bonds.

"Heteroalkynylene" refers to an aliphatic, branched or straight-chain, divalent, hydrocarbon radical having at least one carbon-carbon triple bond and the indicated number of carbon atoms, wherein one or more (*e.g*., 1, 2, 3, 4, 5 or 6) of the carbon atoms in the chain is replaced with a heteroatom selected from N, O, S and Si. Thus, "(C₂-C₂₅)heteroalkynylene" means a diradical having at least one carbon-carbon triple bond and from 2 to 25 carbon atoms in a linear or branched arrangement, wherein one or more (*e.g*., 1, 2, 3, 4, 5 or 6) of the carbon atoms in the chain is replaced with a heteroatom selected from N, O, S and Si. In some embodiments, the alkynylene group has one, two, or three carbon-carbon triple bonds.

"Hydroxyl" refers to -OH.

"Hydroxyl protecting group" refers to a chemical group that replaces the hydrogen of a hydroxyl group and decreases the reactivity of the oxygen atom of the hydroxyl group under at least certain synthetic conditions. Examples of hydroxyl protecting groups can be found, for example, in Wuts, P.G.M. Protecting Groups in Organic Synthesis, 5th Ed., New York, John Wiley & Sons, 2014, and include silyl protecting groups and triisopropylsilyloxymethyl (TOM). Examples of protected hydroxyl groups include, but are not limited to, esters, carbonates, sulfonates allyl ethers, ethers, silyl ethers, alkyl ethers, arylalkyl ethers, and alkoxyalkyl ethers. Examples of suitable esters include formates, acetates, proprionates, pentanoates, crotonates, and benzoates. Specific examples of suitable esters include formate, benzoyl formate, chloroacetate, trifluoroacetate, methoxyacetate, triphenylmethoxyacetate, p-chlorophenoxyacetate, 3-phenylpropionate, 4-oxopentanoate, 4,4-(ethylenedithio)pentanoate, pivaloate (trimethylacetate), crotonate, 4-methoxy-crotonate, benzoate, p-benylbenzoate, 2,4,6-trimethylbenzoate. Examples of carbonates include 9-fluorenylmethyl, ethyl, 2,2,2-trichloroethyl, 2-(trimethylsilyl)ethyl, 2-(phenylsulfonyl)ethyl, vinyl, allyl, and p-nitrobenzyl carbonate. Examples of silyl ethers include trimethylsilyl, triethylsilyl, t-butyldimethylsilyl, t-butyldiphenylsilyl, triisopropylsilyl ether, and other trialkylsilyl ethers. Examples of alkyl ethers include methyl, benzyl, p-methoxybenzyl, 3,4-dimethoxybenzyl, trityl, t-butyl, and allyl ether, or derivatives thereof. Alkoxyalkyl ethers include acetals such as methoxymethyl, methylthiomethyl, (2-methoxyethoxy)methyl, benzyloxymethyl, beta-(trimethylsilyl)ethoxymethyl, and tetrahydropyran-2-yl ether. Examples of arylalkyl ethers include benzyl, p-methoxybenzyl (MPM), 3,4-dimethoxybenzyl, O-nitrobenzyl, p-nitrobenzyl, p-halobenzyl, 2,6-dichlorobenzyl, p-cyanobenzyl, 2- and 4-picolyl ethers. In some embodiments, the hydroxyl protecting group is an acid-labile hydroxy protecting group, meaning that it can be removed under acidic conditions.

When the term "substituted" precedes a designated group, it means that one or more hydrogens of the designated group are replaced with a suitable substituent. Unless otherwise indicated, an "optionally substituted" group or "substituted or unsubstituted" group may be substituted or unsubstituted. When an "optionally substituted" group or "substituted or unsubstituted" group is substituted, the group can have a suitable substituent at each substitutable position of the group and, when more than one position in any given structure may be substituted with more than one substituent selected from a specified group, the substituent can be the same or different at every position. Alternatively, an "optionally substituted" group or "substituted or unsubstituted" group can be unsubstituted. A designated group can be substituted or unsubstituted, but typically, a designated group is unsubstituted, unless otherwise indicated, e.g., by provision of a variable that denotes allowable substituents for a designated group. For example, R⁵ in Structural Formula II denotes allowable substituents for the phenyl of variable R⁴. However, any of the heteroalkyl, heteroalkylene, heteroalkenyl, heteroalkenylene, heteroalkynyl and heteroalkynylene groups described herein can be unsubstituted or substituted with one or more (*e.g*., 1, 2, 3, 4, 5 or 6, such as 1, 2 or 3) oxo (=O), imino (=N(H) or =N(aliphatic) or =N(heteroaliphatic)) or thio (=S) groups. In some embodiments, a heteroalkyl, heteroalkylene, heteroalkenyl, heteroalkenylene, heteroalkynyl or heteroalkynylene is unsubstituted. In some embodiments, a heteroalkyl, heteroalkylene, heteroalkenyl, heteroalkenylene, heteroalkynyl or heteroalkynylene is substituted with one or more (*e.g*., 1, 2 3, 4 or 5, such as 1, 2 or 3) oxo.

Suitable monovalent substituents of an optionally substituted group or substituted or unsubstituted group (*e.g*., aryl) include halo, (C₁-C₅)alkyl, (C₁-C₅)haloalkyl, (C₁-C₅)alkoxy and (C₁-C₅)haloalkoxy. Suitable divalent substituents of an optionally substituted group or substituted or unsubstituted group include oxo (=O), imino (=N(H) or =N(aliphatic) or =N(heteroaliphatic)) and thio (=S) groups (*e.g*., oxo).

Groups described herein having two or more points of attachment (*i.e.,* divalent, trivalent, or polyvalent) within the compound of the present technology are designated by use of the suffix, "ene." For example, divalent alkyl groups are alkylene groups, divalent heteroalkyl groups are heteroalkylene groups, and so forth. Substituted groups having a single point of attachment to the compound of the present technology are not referred to using the "ene" designation. Thus, e.g., chloroethyl is not referred to herein as chloroethylene.

"Silyl protecting group" refers to a protecting group of the general formula - Si(R°)₃, wherein each R° is independently (C₁-C₁₀)alkyl or (C₆-C₁₂)aryl. Examples of silyl protecting groups can be found, for example, in Wuts, P.G.M. Protecting Groups in Organic Synthesis, 5th Ed., New York, John Wiley & Sons, 2014, and include *tert*-butyldimethylsilyl (TBDMS), trimethylsilyl (TMS), triethylsilyl (TES), triisopropylsilyl (TIPS) and *tert-*butyldiphenylsilyl (TBDPS). Silyl protecting groups are typically acid-labile or removed by fluoride ion. Silyl protecting groups are commonly used to protect hydroxyl and thiol functional groups.

"Silylating agent" refers to a silyl-containing chemical agent that reacts with a functional group, such as a hydroxyl or thiol, to produce a silylated derivative of that functional group. Typically, the silyl-containing portion of the silylated derivative produced upon reaction of a silylating agent and a functional group can be removed with an appropriate deprotecting agent, such as an acid or fluoride ion. Examples of silylating agents can be found, for example, in Wuts, P.G.M. Protecting Groups in Organic Synthesis, 5th Ed., New York, John Wiley & Sons, 2014, and include TBDMS-Cl, TMSCl, TESCl, TIPSCl, TBDPSCl, *etc.*

"Thio," "thiol" and "sulfhydryl" refer to -SH.

"Thioalkoxy" refers to the -S-alkyl radical, wherein alkyl is as described herein.

"Thiol protecting group" or "sulfhydryl protecting group" refers to a chemical group that replaces the hydrogen atom of a thiol group and decreases the reactivity of the sulfur atom of the thiol group under at least certain synthetic conditions. Examples of thiol protecting groups can be found, for example, in Wuts, P.G.M. Protecting Groups in Organic Synthesis, 5th Ed., New York, John Wiley & Sons, 2014. Protected thiols include disulfides, thioethers (*e.g*., alkylthioethers, benzyl and substituted benzyl thioethers, triphenylmethyl thioethers), silyl thioethers, thioesters (*e.g*., trichloroethoxycarbonyl thioester), thiocarbonates, thiocarbamates, and the like. In some embodiments, the thiol protecting group is an acid-labile thiol protecting groups.

Structures depicted herein (*e.g*., Structural Formulas (Ia)-(Ii), (II), (III)) can be present in free base form or as salts, e.g., salts derived from inorganic or organic acids ("acid addition salts"), or inorganic or organic bases ("base addition salts"). Examples of acid addition salts are salts of an amino group formed with inorganic acids such as hydrochloric acid, hydrobromic acid, phosphoric acid, sulfuric acid and perchloric acid, or with organic acids such as acetic acid, oxalic acid, maleic acid, tartaric acid, citric acid, succinic acid or malonic acid or by using other methods used in the art, such as ion exchange. Other acid addition salts include adipate, alginate, ascorbate, aspartate, benzenesulfonate, benzoate, bisulfate, borate, butyrate, camphorate, camphorsulfonate, cinnamate, citrate, cyclopentanepropionate, digluconate, dodecylsulfate, ethanesulfonate, formate, fumarate, glucoheptonate, glycerophosphate, gluconate, glutarate, glycolate, hemisulfate, heptanoate, hexanoate, hydroiodide, hydroxybenzoate, 2-hydroxy-ethanesulfonate, hydroxymaleate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 2-phenoxybenzoate, phenylacetate, 3-phenylpropionate, phosphate, pivalate, propionate, pyruvate, salicylate, stearate, succinate, sulfate, tartrate, thiocyanate, p-toluenesulfonate, undecanoate, valerate salts, and the like. Either the mono-, di- or tri-acid salts can be formed, and such salts can exist in either a hydrated, solvated or substantially anhydrous form.

Base addition salts include salts derived from inorganic bases, such as alkali metal, alkaline earth metal, and ammonium bases, and salts derived from aliphatic, alicyclic or aromatic organic amines, such as methylamine, trimethylamine, triethylamine and picoline, or N⁺((C₁-C₄)alkyl)₄ salts. Representative alkali or alkaline earth metal salts include sodium, lithium, potassium, calcium, magnesium, barium and the like. Further base addition salts include ammonium, quaternary ammonium, and amine cations formed using counterions such as halide, hydroxide, carboxyl, sulfate, phosphate, nitrate, lower alkyl sulfonate and aryl sulfonate.

Additionally, unless otherwise stated, structures depicted herein are also meant to include compounds that differ only in the presence of one or more isotopically enriched atoms. For example, compounds produced by the replacement of a hydrogen with deuterium or tritium, or of a carbon with a ¹³C- or ¹⁴C-enriched carbon or of a phosphorus with a ³²P-enriched phosphorus are within the scope of this disclosure. Such compounds are useful, for example, as analytical tools, as probes in biological assays, or as therapeutic agents. Procedures for inserting such labels into the compounds of the present technology will be readily apparent to those skilled in the art based on the disclosure herein.

Those of skill in the art will appreciate that compounds of the present technology may exhibit the phenomena of tautomerism, conformational isomerism, geometric isomerism and/or stereoisomerism. Unless indicated otherwise, the present technology encompasses any tautomeric, conformational isomeric, stereochemical and/or geometric isomeric forms of the compounds having one or more of the utilities described herein, as well as mixtures of these various different forms.

Stereoisomers of compounds (also known as optical isomers) include all chiral, diastereomeric, and racemic forms of a structure, unless the specific stereochemistry is expressly indicated. Thus, compounds used in the present technology include enriched or resolved optical isomers at any or all asymmetric atoms as are apparent from the depictions. Both racemic and diastereomeric mixtures, as well as the individual optical isomers may be isolated or synthesized so as to be free or substantially free from their enantiomeric or diastereomeric partners, and these stereoisomers are all within the scope of the present technology.

### Covalently-modified macroporous, controlled porosity silica gel

Provided herein is macroporous, controlled porosity silica gel (CPSG) covalently modified with a moiety suitable for oligonucleotide synthesis. For example, one embodiment provides in accordance with this disclosure provides macroporous CPSG covalently modified with a moiety comprising a spacer, a linker and a nucleoside (*e.g*., a moiety of structural formula la, Ib, Ic, Id, Ie, If, Ig, Ih, Ii). In another embodiment in accordance with this disclosure, macroporous CPSG is covalently modified with a moiety comprising a spacer and a universal linker (*e.g*., a moiety of structural formula II). In yet another embodiment in accordance with this disclosure, macroporous CPSG is covalently modified with a moiety comprising a spacer, a linker and a chromophore, ligand or bioconjugation linker (*e.g*., a moiety of structural formula III).

Silica gel is an amorphous and porous form of silicon dioxide. Silica gel can be made by acidifying a solution of sodium silicate, and washing and drying the resultant precipitate. By controlling conditions such as stirring velocity, rate of acidification and solution viscosity, both particle and pore sizes can be controlled, resulting in controlled porosity silica gel and, if desired, macroporous controlled porosity silica gel. Macroporous CPSG is available in a variety of mean particle sizes (*e.g*., mean particle sizes of greater than or about 2 microns, greater than or about 5 microns, greater than or about 10 microns, greater than or about 20 microns, greater than or about 50 microns or greater than or about 70 microns and/or less than or about 500 microns, less than or about 250 microns, less than or about 200 microns, less than or about 150 microns, for example, from about 50 microns to about 500 microns, from about 70 microns to about 200 microns or from about 70 microns to about 150 microns), mean pore sizes (*e.g*., mean pore sizes of greater than or about 500 Å to about 5,000 Å, greater than or about 500 Å to about 1,000 Å, greater than or about 750 Å to about 1,000 Å, about 800 Å or about 1,000 Å) and particle size distributions (*e.g*., ± 5 standard deviations from the mean particle size, ± 4 standard deviations from the mean particle size, ± 3 standard deviations from the mean particle size, ± 2 standard deviations from the mean particle size, ± 1 standard deviation from the mean particle size, ± 0.5 standard deviations from the mean particle size, ± 0.1 standard deviations from the mean particle size). Macroporous CPSG, *e.g*., with an average pore size between 500 Å and 4,000 Å, can be synthesized in accordance with the procedure disclosed in G.Bruno, F.Gasparrini, D.Misiti, E.Arrigoni-Martelli, M.Bronzetti, Journal of Chromatography A, 1990, 504, 319-333. Macroporous CPSG is also available from Zeochem, ITOCHU Chemicals America, Inc., Supelco, Millipore/Sigma Aldrich, Fuji Silysia Chemical and YMC Europe GmbH.

Particle size analysis can be used to measure particle size and, more typically, the size distribution of particles in a sample. Most particle sizing techniques measure a one-dimensional property of a particle (*e.g*., surface area, volume), and relate the measured property to the size of an equivalent sphere. Particle size can be expressed as a mean of a representative sample, such as a representative number of silica gel particles in the case of silica gel. Methods of measuring particle size are known in the art, and include direct imaging (*e.g*., using a cell counter) and laser diffraction.

In some embodiments, macroporous CPSG (*e.g*., covalently-modified, macroporous CPSG described herein) has a mean particle size of greater than or about 2 microns, greater than or about 5 microns, greater than or about 10 microns, greater than or about 20 microns, greater than or about 50 microns or greater than or about 70 microns. In some embodiments, macroporous CPSG (*e.g*., covalently-modified, macroporous CPSG described herein) has a mean particle size of less than or about 500 microns, less than or about 250 microns, less than or about 200 microns or less than or about 150 microns. Any range formed by combination of two of the aforementioned mean particle sizes is also within the scope of this disclosure. For example, in some embodiments, macroporous CPSG (*e.g*., covalently-modified, macroporous CPSG described herein) has a mean particle size of from about 50 microns to about 500 microns, from about 70 microns to about 250 microns, from about 70 microns to about 200 microns or from about 70 microns to about 150 microns.

Pore size is a measure of the distance between two opposite walls of a pore. It will be understood that, in the case of a circular pore, pore size is the diameter of the pore, while in the case of a non-circular pore, pore size may be, *e.g*., the length or width of the pore at the measured location. Pore size can be expressed as a mean of a representative sample, such as a representative number of pores in one or more silica gel particles in the case of silica gel. Methods of measuring pore size are known in the art, and include gas (*e.g*., argon) adsorption analysis, electron microscopy and differential scanning calorimetry.

In some embodiments, macroporous CPSG (*e.g*., covalently-modified, macroporous CPSG described herein) has a mean pore size of greater than or about 500 Å to about 5,000 Å, greater than or about 500 Å to about 1,000 Å, greater than or about 750 Å to about 1,000 Å, about 800 Å or about 1,000 Å.

Particle size distribution is a means of expressing what sizes of particles in what proportions are present in a population of particles. Methods of measuring particle size distribution are known in the art, and include laser diffraction.

In some embodiments, macroporous CPSG (*e.g*., covalently-modified, macroporous CPSG described herein) has a particle size distribution of ± 5 standard deviations from the mean particle size, ± 4 standard deviations from the mean particle size, ± 3 standard deviations from the mean particle size, ± 2 standard deviations from the mean particle size, ± 1 standard deviation from the mean particle size, ± 0.5 standard deviations from the mean particle size, or ± 0.1 standard deviations from the mean particle size.

Macroporous CPSG is available in a variety of densities. In some embodiments, macroporous CPSG has a density of from about 0.35 g/mL to about 0.75 g/mL. In more specific embodiments, macroporous CPSG has a density of from about 0.4 g/mL to about 0.6 g/mL. In yet more specific embodiments, macroporous CPSG has a density of about 0.5 g/mL.

As with all solid supports for oligonucleotide synthesis, the amount of derivatization on the support determines the maximum amount of oligonucleotide that can ultimately by prepared using the solid support. The amount of derivatization can be referred to as loading, and can be expressed as a fraction of the molar quantity (*e.g*., micromoles) of the moiety(ies) covalently attached to the macroporous CPSG over the weight (*e.g*., grams) of the covalently-modified, macroporous CPSG. The covalently-modified, macroporous CPSG described herein typically contains less than or about 150 (*e.g*., 100) micromoles of the moiety suitable for oligonucleotide synthesis (*e.g*., the moiety of any of structural formulas la, Ib, Ic, II and III) per gram of the covalently-modified, macroporous CPSG, such as from about 25 micromoles of the moiety per gram of the covalently-modified, macroporous CPSG to about 100 micromoles of the moiety per gram of the covalently-modified, macroporous CPSG, from about 35 micromoles of the moiety per gram of the covalently-modified, macroporous CPSG to about 80 micromoles of the moiety per gram of the covalently-modified, macroporous CPSG or from about 40 micromoles of the moiety per gram of the covalently-modified, macroporous CPSG to about 75 micromoles of the moiety per gram of the covalently-modified, macroporous CPSG. Methods of measuring loading are known in the art, and include ultraviolet-visible spectroscopy, *e.g*., of the 4,4'-dimethoxytrityl group released from an oligonucleotide upon deprotection of the 5'-hydroxy functional group.

The chemical group(s) that join the solid support to the first nucleoside generally include a spacer and a linker. The spacer connects the solid support, such as macroporous CPSG, to the linker, and generally terminates in a nucleophilic functional group (*e.g*., amino, hydroxyl, thio) that can be used to facilitate attachment of the linker to the spacer. Examples of spacers include (R¹)₂Si-(C₁-C₂₅)alkylene-Z^, (R¹)₂Si-(C₂-C₂₅)alkenylene-Z^, (R¹)₂Si-(C₂-C₂₅)alkynylene-Z^, (R¹)₂Si-(C₁-C₂₅)heteroalkylene-Z^, (R¹)₂Si-(C₂-C₂₅)heteroalkenylene-Z^ and (R¹)₂Si-(C₂-C₂₅)heteroalkynylene-Z^, wherein ^ indicates the point of attachment of the spacer to the linker; each R¹ is independently an additional point of attachment of the spacer to the macroporous CPSG, or -O(C₁-C₁₀)alkyl or -O(C₆-C₁₂)aryl; and Z is N(H), O or S. Other examples of spacers include that derived from aminopropyl, long-chain alkyl amines derived from amino(C₆-C₁₂)alkanoyl-modified aminopropyl (*e.g*., aminocaproic acid-modified aminopropyl), and the long-chain alkyl amine spacer disclosed in Richard T. Pon, Current Protocols in Nucleic Acid Chemistry (2000) 3.1.1-3.1.28. Further examples of spacers include those disclosed in Richard T. Pon, Current Protocols in Nucleic Acid Chemistry (2000) 3.1.1-3.1.28, the entire content of which is incorporated herein by reference.

The linker is a difunctional chemical group that connects the spacer to a first nucleoside unit, either directly, as in the moieties represented by structural formulas Ia-Ii, or indirectly (*e.g*., via a chromophore, ligand or bioconjugation linker), as in the moiety represented by structural formula III, and can be cleaved, upon completion of a solid-phase oligonucleotide synthesis based on the solid support, to produce a free oligonucleotide. Examples of linkers include universal linkers and succinyl, oxalyl, malonyl, diglycolyl and hydroquinone-*O*,*O*'-diacetyl groups. Further examples of linkers include those disclosed in Richard T. Pon, Current Protocols in Nucleic Acid Chemistry (2000) 3.1.1-3.1.28, the entire content of which is incorporated herein by reference.

Universal linkers are specialized linkers developed to enable the terminal nucleoside of an oligonucleotide to be added as part of an automated synthesis, and can be used to produce oligonucleotides with free, 3'-OH ends. A universal linker (such as the universal linker shown in the moiety of structural formula II; UnyLinker™, available from ChemGenes Corporation, Wilmington, MA) can be attached to a solid support via a spacer, or can be attached to a solid support via a spacer and another linker (as in the moiety represented by structural formula II). Examples of universal linkers include that depicted in structural formula II. Further examples of universal linkers include those disclosed in Richard T. Pon, Current Protocols in Nucleic Acid Chemistry (2000) 3.1.1-3.1.28, the entire content of which is incorporated herein by reference.

Nucleosides are typically made up of a five-carbon sugar ribose radical or a derivative thereof covalently attached to a nucleoside base radical or modified nucleoside base radical at the 1'-position of the ribose. However, in some embodiments, a nucleoside lacks a nucleoside base radical or modified nucleoside base radical. In such embodiments, the ribose radical of the nucleoside is attached to a hydrogen radical at the 1'-position of the ribose. Nucleosides lacking a nucleoside base radical or modified nucleoside base radical are exemplified by moieties of structural formulas Ia-Ii wherein B is H.

Examples of nucleoside base radicals include 9-(N⁶-benzoyladeninyl)-, 9-(N⁶-( N,N-dimethylformamidinyl)-adeninyl), 1-(N⁴-acetylcytosinyl)-, 1-(N⁴-benzoylcytosinyl)-, 1-(N⁴-isobutyryl cytosinyl)-, 1-(N⁴-(N,N-dimethylformamidinyl)cytosinyl)-, 1-(N⁴-phenoxyacetylcytosinyl)-, 1-(N⁴-*tert*-butylphenoxyacetylcytosinyl)-, 1-(N⁴-isopropyl phenoxyacetylcytosinyl)-, 9-(N²-isobutyrylguaninyl)-,9-(N²-*tert-*butylphenoxyacetylguaninyl)-,9-(N²-isopropylphenoxyacetylguaninyl)-, 9-(N⁶-(N,N-dimethylformamidinyl)-guaninyl)-, 1-uracilyl- and pseudouracilyl.

Examples of modified nucleoside base radicals include 1-(N⁴-benzoyl-5-methylcytosinyl)-, 1-(N⁴-(N,N-dimethylformamidinyl)-5-methylcytosinyl)-, 1-(N⁴-acetyl-5-methylcytosinyl)-, 1-(5-methyl-uracilyl)-, 1-(5-fluoro-uracilyl)-, 1-(N⁴-benzoyl-5-fluorocytosinyl)-, 9-(N⁶-benzoyl-7-deazaadeninyl)-, 9-(N⁶-(N,N-dimethylformamidinyl)-7-deazaadeninyl)-, 9-(N²-isobutyryl-7-deazaguaninyl)-, 9-(N²-(N,N-dimethylformamidinyl)-7-deazaguaninyl)-, 1-(N⁴-benzoyl-5-bromo-cytosinyl)-, 1-(5-bromo-uracilyl)-, 1-(5-iodo-uracilyl)-, 1-(5-vinyl-uracilyl)-, 1-(N³-methyl-uracilyl)-, 1-(N⁴-benzoyl-N³-methylcytosinyl)-, 1-(N³-methyl-5-methyluracilyl)-, 9-purinyl, 9-(N²-phenoxyacetyl-2-aminopurinyl)-, 9-(N^{2,} N⁶-diphenoxyacetyl-2,6-diaminopurinyl)-, 9-(N⁶-benzoyl-8-bromoadeninyl)-, 9-(N⁶-benzoyl-8-oxoadeninyl)-, 9-(N¹-methyl-N⁶-(9-fluorenylmethyloxycarbonyl)-7-deazaadeninyl)-, 9-(N²-isobutyryl-8-oxoguaninyl)-, 9-(N⁶-(N,N-dimethylformamidinyl)-8-oxoguaninyl)-, 9-(etheno-adeninyl)-, 1-(etheno-cytosinyl)-, 9-(hypoxanthinyl)-, 9-(8-bromohypoxanthinyl)-, 9-(N²-methylhypoxanthinyl)-, 5-(1,2-diacetyloxyethyl)-uracilyl, *N*³-acetyl-5-(1,2-diacetyloxyethyl)-cytosinyl, 5-acetoxymethyluracilyl and *N*³-acetyl-5-cyanoethoxymethyl-cytosinyl.

Nucleosides (*e.g*., the first nucleoside) joinable to the solid support via a spacer and a linker, and nucleosides in a moiety comprising a spacer, a linker and a nucleoside in accordance with this disclosure include D-(β)-nucleosides (*e.g*., as in the moieties represented by structural formulas la, Ib and Ic), arabino nucleosides (e.g., as in the moiety represented by structural formula Id), L-(β)-nucleosides (*e.g*., as in the moiety represented by structural formula Ie), D-(α)-nucleosides (*e.g*., as in the moiety represented by structural formula If), locked nucleic acids (LNA) (*e.g*., as in the moiety represented by structural formula Ig), 2'-O,4'-C-ethylene-bridged nucleic acids (ENA) (*e.g*., as in the moiety represented by structural formula Ih) and bridged nucleic acids (BNA) (*e.g*., as in the moiety represented by structural formula Ii).

In a first embodiment, macroporous CPSG is covalently modified with a moiety represented by one of the following structural formulas: 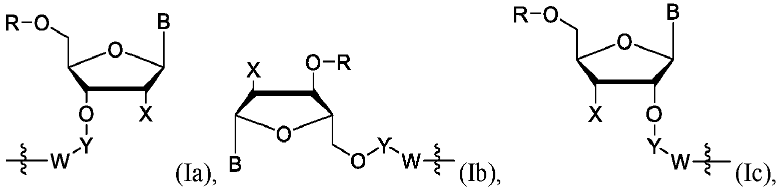 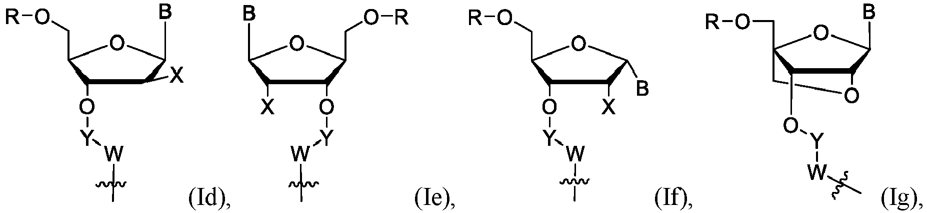 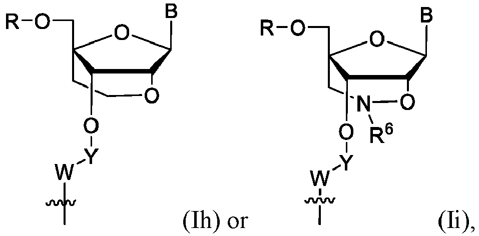 wherein:
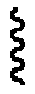 indicates the point of attachment of the moiety to macroporous CPSG;
B is a nucleoside base radical (*e.g*., selected from 9-(N⁶-benzoyladeninyl)-, 9-(N⁶-( N,N-dimethylformamidinyl)-adeninyl), 1-(N⁴-acetylcytosinyl)-, 1-(N⁴-benzoylcytosinyl)-, 1-(N⁴-isobutyrylcytosinyl)-, 1-(N⁴-(N,N-dimethylformamidinyl)cytosinyl)-, 1-(N⁴-phenoxyacetylcytosinyl)-, 1-(N⁴*-tert-*butylphenoxyacetylcytosinyl)-, 1-(N⁴-isopropyl phenoxyacetylcytosinyl)-, 9-(N²-isobutyrylguaninyl)-,9-(N²-*tert*-butylphenoxyacetylguaninyl)-,9-(N²-isopropylphenoxyacetylguaninyl)-, 9-(N⁶-(N,N-dimethylformamidinyl)-guaninyl)-, 1-uracilyl- or pseudouracilyl); a modified nucleoside base radical (*e.g*., selected from 1-(N⁴-benzoyl-5-methylcytosinyl)-, 1-(N⁴-(N,N-dimethylformamidinyl)-5-methylcytosinyl)-, 1-(N⁴-acetyl-5-methylcytosinyl)-, 1-(5-methyl-uracilyl)-, 1-(5-fluoro-uracilyl)-, 1-(N⁴-benzoyl-5-fluorocytosinyl)-, 9-(N⁶-benzoyl-7-deazaadeninyl)-, 9-(N⁶-(N,N-dimethylformamidinyl)-7-deazaadeninyl)-, 9-(N²-isobutyryl-7-deazaguaninyl)-, 9-(N²-(N,N-dimethylformamidinyl)-7-deazaguaninyl)-, 1-(N⁴-benzoyl-5-bromo-cytosinyl)-, 1-(5-bromo-uracilyl)-, 1-(5-iodo-uracilyl)-, 1-(5-vinyl-uracilyl)-, 1-(N³-methyl-uracilyl)-, 1-(N⁴-benzoyl-N³-methylcytosinyl)-, 1-(N³-methyl-5-methyluracilyl)-, 9-purinyl, 9-(N²-phenoxyacetyl-2-aminopurinyl)-, 9-(N^{2,}N⁶-diphenoxyacetyl-2,6-diaminopurinyl)-, 9-(N⁶-benzoyl-8-bromoadeninyl)-, 9-(N⁶ -benzoyl-8-oxoadeninyl)-, 9-(N¹-methyl-N⁶-(9-fluorenylmethyloxycarbonyl)-7-deazaadeninyl)-, 9-(N²-isobutyryl-8-oxoguaninyl)-, 9-(N⁶-(N,N-dimethylformamidinyl)-8-oxoguaninyl)-, 9-(etheno-adeninyl)-, 1-(etheno-cytosinyl)-, 9-(hypoxanthinyl)-, 9-(8-bromohypoxanthinyl)-, 9-(N²-methylhypoxanthinyl)-, 5-(1,2-diacetyloxyethyl)-uracilyl, *N*³-acetyl-5-(1,2-diacetyloxyethyl)-cytosinyl, 5-acetoxymethyluracilyl or *N*³-acetyl-5-cyanoethoxymethyl-cytosinyl); or H;
R is H or 4,4'-dimethoxytrityl;
R⁶ is methyl or acetyl;
W is a spacer;
X is hydrogen, halogen *(e.g.,* fluoro), hydroxyl, OP *(e.g.,* -OCH₂CH₂OCH₃, -OCH₂CH=CH₂ or -OCH₂C≡CH), thio, (C₁-C₁₀)alkoxy (*e.g*., -OCH₃, -OCH₂CH₃), (C₁-C₁₀)thioalkoxy, NH₂, N(H)P³ or NP³₂ *(e.g.,* hydrogen, halogen *(e.g.,* fluoro), hydroxyl, OP (*e.g*., -OCH₂CH₂OCH₃, -OCH₂CH=CH₂ or -OCH₂C≡CH), or (Ci-C₁₀)alkoxy (*e.g*., -OCH₃, -OCH₂CH₃));
P is -OCH₂CH₂OCH₃, -OCH₂CH=CH₂ or -OCH₂C≡CH, or a hydroxyl protecting group (*e.g*., *tert*-butyldimethylsilyl, triisopropylsilyloxymethyl (TOM));
each P³ is independently an amino protecting group or two P³, taken together with the nitrogen to which they are attached, form a cyclic di-protected amino; and
Y is a linker (*e.g*., succinyl, oxalyl, malonyl, diglycolyl or hydroquinone-*O,O'-*diacetyl).

In a first aspect of the first embodiment:
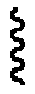 indicates the point of attachment of the moiety to macroporous CPSG;
B is a nucleoside base radical selected from 9-(N⁶-benzoyladeninyl)-, 9-(N⁶-(N,N-dimethylformamidinyl)-adeninyl), 1-(N⁴-acetylcytosinyl)-, 1-(N⁴-benzoylcytosinyl)-, 1-(N⁴-isobutyrylcytosinyl)-,1-(N⁴-(N,N-dimethylformamidinyl)cytosinyl)-, 1-(N⁴-phenoxyacetylcytosinyl)-, 1-(N⁴*-tert-*butylphenoxyacetylcytosinyl)-, 1-(N⁴-isopropyl phenoxyacetylcytosinyl)-, 9-(N²-isobutyrylguaninyl)-,9-(N²-*tert*-butylphenoxyacetylguaninyl)-,9-(N²-isopropylphenoxyacetylguaninyl)-, 9-(N⁶-(N,N-dimethylformamidinyl)-guaninyl)-, 1-uracilyl- or pseudouracilyl; a modified nucleoside base radical selected from 1-(N⁴-benzoyl-5-methylcytosinyl)-, 1-(N⁴-(N,N-dimethylformamidinyl)-5-methylcytosinyl)-, 1-(N⁴-acetyl-5-methylcytosinyl)-, 1-(5-methyl-uracilyl)-, 1-(5-fluoro-uracilyl)-, 1-(N⁴-benzoyl-5-fluorocytosinyl)-, 9-(N⁶-benzoyl-7-deazaadeninyl)-, 9-(N⁶-(N,N-dimethylformamidinyl)-7-deazaadeninyl)-, 9-(N²-isobutyryl-7-deazaguaninyl)-, 9-(N²-(N,N-dimethylformamidinyl)-7-deazaguaninyl)-, 1-(N⁴-benzoyl-5-bromo-cytosinyl)-, 1-(5-bromo-uracilyl)-, 1-(5-iodo-uracilyl)-, 1-(5-vinyl-uracilyl)-, 1-(N³-methyl-uracilyl)-, 1-(N⁴-benzoyl-N³-methylcytosinyl)-, 1-(N³-methyl-5-methyluracilyl)-, 9-purinyl, 9-(N²-phenoxyacetyl-2-aminopurinyl)-, 9-(N^{2,}N⁶-diphenoxyacetyl-2,6-diaminopurinyl)-, 9-(N⁶-benzoyl-8-bromoadeninyl)-, 9-(N⁶ -benzoyl-8-oxoadeninyl)-, 9-(N¹-methyl-N⁶-(9-fluorenylmethyloxycarbonyl)-7-deazaadeninyl)-, 9-(N²-isobutyryl-8-oxoguaninyl)-, 9-(N⁶-(N,N-dimethylformamidinyl)-8-oxoguaninyl)-, 9-(etheno-adeninyl)-, 1-(etheno-cytosinyl)-, 9-(hypoxanthinyl)-, 9-(8-bromohypoxanthinyl)-, 9-(N²-methylhypoxanthinyl)-, 5-(1,2-diacetyloxyethyl)-uracilyl, *N*³-acetyl-5-(1,2-diacetyloxyethyl)-cytosinyl, 5-acetoxymethyluracilyl or *N*³-acetyl-5-cyanoethoxymethyl-cytosinyl; or H;
R is H or 4,4'-dimethoxytrityl;
W is a spacer selected from (R¹)₂Si-(C₁-C₂₅)alkylene-Z^, (R¹)₂Si-(C₂-C₂₅)alkenylene-Z^, (R¹)₂Si-(C₂-C₂₅ )alkynylene-Z^, (R¹)₂Si-(C₁-C₂₅)heteroalkylene-Z^, (R¹)₂Si-(C₂-C₂₅)heteroalkenylene-Z^ or (R¹)₂Si-(C₂-C₂₅)heteroalkynylene-Z^;
^ indicates the point of attachment of W to Y;
each R¹ is independently an additional point of attachment of W to the macroporous CPSG, or -O(C₁-C₁₀)alkyl or -O(C₆-C₁₂)aryl;
R⁶ is methyl or acetyl;
X is hydrogen, halogen (*e.g.,* fluoro), hydroxyl, OP (*e.g.,* -OCH₂CH₂OCH₃, -OCH₂CH=CH₂ or -OCH₂C≡CH), thio, (C₁-C₁₀)alkoxy (*e.g*., -OCH₃, -OCH₂CH₃), (C₁-C₁₀)thioalkoxy, NH₂, N(H)P³ or NP³₂ (*e.g*., hydrogen, halogen (*e.g*., fluoro), hydroxyl, OP (*e.g*., -OCH₂CH₂OCH₃, -OCH₂CH=CH₂ or -OCH₂C≡CH), or (Ci-C₁₀)alkoxy (*e.g*., -OCH₃, -OCH₂CH₃));
P is -OCH₂CH₂OCH₃, -OCH₂CH=CH₂ or -OCH₂C≡CH, or a hydroxyl protecting group (*e.g*., *tert*-butyldimethylsilyl, triisopropylsilyloxymethyl (TOM));
each P³ is independently an amino protecting group or two P³, taken together with the nitrogen to which they are attached, form a cyclic di-protected amino;
Y is a linker selected from succinyl, oxalyl, malonyl, diglycolyl or hydroquinone-*O,O'*-diacetyl; and
Z is N(H), O or S (*e.g*., N(H)).

In a second aspect of the first embodiment, the moiety is represented by structural formula la, wherein values for the variables are as described in the first embodiment, or first aspect thereof.

In a third aspect of the first embodiment, the moiety is represented by structural formula Ib, wherein values for the variables are as described in the first embodiment, or first aspect thereof.

In a fourth aspect of the first embodiment, the moiety is represented by structural formula Ic, wherein values for the variables are as described in the first embodiment, or first aspect thereof.

In a fifth aspect of the first embodiment, the moiety is represented by structural formula Id, wherein values for the variables are as described in the first embodiment, or first aspect thereof.

In a sixth aspect of the first embodiment, the moiety is represented by structural formula Ie, wherein values for the variables are as described in the first embodiment, or first aspect thereof.

In a seventh aspect of the first embodiment, the moiety is represented by structural formula If, wherein values for the variables are as described in the first embodiment, or first aspect thereof.

In an eighth aspect of the first embodiment, the moiety is represented by structural formula Ig, wherein values for the variables are as described in the first embodiment, or first aspect thereof.

In a ninth aspect of the first embodiment, the moiety is represented by structural formula Ih, wherein values for the variables are as described in the first embodiment, or first aspect thereof.

In a tenth aspect of the first embodiment, the moiety is represented by structural formula Ii, wherein values for the variables are as described in the first embodiment, or first aspect thereof.

In an eleventh aspect of the first embodiment, X is hydrogen, halogen (*e.g*., fluoro), hydroxyl, -OCH₂CH₂OCH₃, -OCH₂CH=CH₂, -OCH₂C≡CH, -OCH₃, -OCH₂CH₃, -O-*tert*-butyldimethylsilyl or -O-TOM. Values for the remaining variables are as described in the first embodiment, or first through tenth aspects thereof.

In a second embodiment, macroporous CPSG is covalently modified with a moiety represented by the following structural formula: 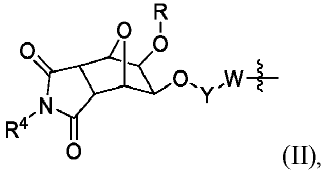 wherein:
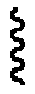 indicates the point of attachment of the moiety to macroporous CPSG;
R is H or 4,4'-dimethoxytrityl;
R⁴ is (C₁-C₂₅)alkyl (*e.g*., methyl, ethyl), or phenyl optionally substituted with one or more (*e.g*., one, two, three) R⁵ (*e.g*., phenyl optionally substituted with one R⁵ at the para position);
R⁵, for each occurrence, is independently halo, (C₁-C₅)alkyl (*e.g*., methyl, such as para-methyl), (C₁-C₅)haloalkyl (*e.g*., trifluoromethyl, such as para-trifluoromethyl), (C₁-C₅)alkoxy (*e.g*., methoxy, such as para-methoxy) or (Ci-C₅)haloalkoxy (*e.g*., trifluoromethoxy, such as para-trifluoromethoxy);
W is a spacer; and
Y is a linker (*e.g*., succinyl, oxalyl, malonyl, diglycolyl or hydroquinone-*O,O'-*diacetyl).

In a first aspect of the second embodiment:
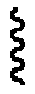 indicates the point of attachment of the moiety to macroporous CPSG;
R is H or 4,4'-dimethoxytrityl;
W is a spacer selected from (R¹)₂Si-(C₁-C₂₅)alkylene-Z^, (R¹)₂Si-(C₂-C₂₅)alkenylene-Z^, (R¹)₂Si-(C₂-C₂₅)alkynylene-Z^, (R¹)₂Si-(C₁-C₂₅)heteroalkylene-Z^, (R¹)₂Si-(C₂-C₂₅)heteroalkenylene-Z^ or (R¹)₂Si-(C₂-C₂₅)heteroalkynylene-Z^;
^ indicates the point of attachment of W to Y;
each R¹ is independently an additional point of attachment of W to the macroporous CPSG, or -O(C₁-C₁₀)alkyl or -O(C₆-C₁₂)aryl;
R⁴ is (C₁-C₂₅)alkyl (*e.g*., methyl, ethyl), or phenyl optionally substituted with one or more *(e.g.,* one, two, three) R⁵ *(e.g.,* phenyl optionally substituted with one R⁵ at the para position);
R⁵, for each occurrence, is independently halo, (C₁-C₅)alkyl (*e.g*., methyl, such as para-methyl), (C₁-C₅)haloalkyl (*e.g*., trifluoromethyl, such as para-trifluoromethyl), (C₁-C₅)alkoxy (*e.g*., methoxy, such as para-methoxy) or (Ci-C₅)haloalkoxy (*e.g*., trifluoromethoxy, such as para-trifluoromethoxy);
Y is a linker selected from succinyl, oxalyl, malonyl, diglycolyl or hydroquinone-*O,O'*-diacetyl; and
Z is N(H), O or S *(e.g.,* N(H)).

In a third embodiment, macroporous CPSG is covalently modified with one or more moieties, each independently represented by the following structural formula: 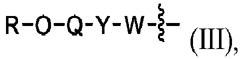 wherein:
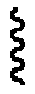 indicates the point of attachment of each moiety to macroporous CPSG;
Q comprises (*e.g*., is) a chromophore *(e.g.,* fluorescein, carboxytetramethylrhodamine (TAMRA), rhodamine X (ROX), sulforhodamine 101 acid chloride (Texas Red), Cy3, Cy5, dabcyl, IQ2 or IQ4); a ligand (*e.g*., cholesterol, tocopherol, palmitic acid, biotin or psoralen); or a bioconjugation linker (*e.g*., a bioconjugation linker covalently attached to Y by N(H), S, O or a dithiolane or dioxalane of one of the
following structural formulas: 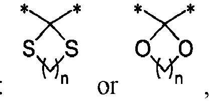 wherein n is 1, 2, 3 or 4 and * indicates the points of attachment of the dithiolane or dioxalane to O and Y);
R is H or 4,4'-dimethoxytrityl;
W is a spacer; and
Y is a linker (*e.g*., succinyl, oxalyl, malonyl, diglycolyl or hydroquinone-*O,O'-*diacetyl).

In a first aspect of the third embodiment:
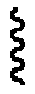 indicates the point of attachment of each moiety to macroporous CPSG;
Q comprises (*e.g*., is) a chromophore (*e.g*., fluorescein, carboxytetramethylrhodamine (TAMRA), rhodamine X (ROX), sulforhodamine 101 acid chloride (Texas Red), Cy3, Cy5, dabcyl, IQ2 or IQ4); a ligand (*e.g*., cholesterol, tocopherol, palmitic acid, biotin or psoralen); or a bioconjugation linker (*e.g*., a bioconjugation linker covalently attached to Y by N(H), S, O or a dithiolane or dioxalane of one of the following structural formulas: 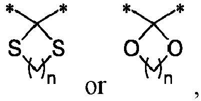 wherein n is 1, 2, 3 or 4 and * indicates the points of attachment of the dithiolane or dioxalane to O and Y);
R is H or 4,4'-dimethoxytrityl;
W is a spacer selected from (R¹)₂Si-(C₁-C₂₅)alkylene-Z^, (R¹)₂Si-(C₂-C₂₅)alkenylene-Z^, (R¹)₂Si-(C₂-C₂₅ )alkynylene-Z^, (R¹)₂Si-(C₁-C₂₅)heteroalkylene-Z^, (R¹)₂Si-(C₂-C₂₅)heteroalkenylene-Z^ or (R¹)₂Si-(C₂-C₂₅)heteroalkynylene-Z^;
^ indicates the point of attachment of W to Y;
each R¹ is independently an additional point of attachment of W to the macroporous CPSG, or -O(C₁-C₁₀)alkyl or -O(C₆-C₁₂)aryl;
Y is a linker selected from succinyl, oxalyl, malonyl, diglycolyl or hydroquinone-*O,O'*-diacetyl; and
Z is N(H), O or S (*e.g*., N(H)).

As used herein, "chromophore" refers to an atom or group of atoms that absorbs light (*e.g*., visible, ultraviolet, infrared light). Examples of chromophores include fluorescein, carboxytetramethylrhodamine (TAMRA), rhodamine X (ROX), sulforhodamine 101 acid chloride (Texas Red), Cy3, Cy5, dabcyl, IQ2 (Part No. N-2010, ChemGenes Corporation, Wilmington, MA) and IQ4 (Part No. N-2000, ChemGenes Corporation, Wilmington, MA).

IQ2 corresponds to a chromophore of the following structure: 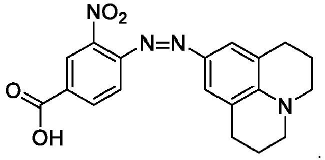 IQ4 corresponds to a chromophore of the following structure: 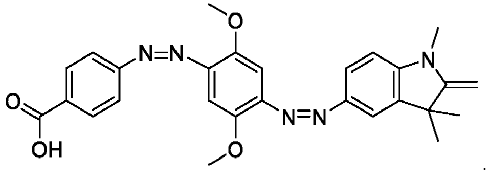 Methods of synthesizing IQ2 and IQ4 and derivatizing the structures of IQ2 and IQ4 for incorporation into a solid support suitable for oligonucleotide synthesis are described in U.S. Patent No. 7,956,169, the entire content of which is incorporated herein by reference.

"Ligand," as used herein, refers to an ion or molecule that forms a complex with another molecule, such as a protein, often by binding to the other molecule. Examples of ligands include cholesterol, tocopherol, palmitic acid, biotin (which binds to avidin/streptavidin) or psoralen (which intercalates DNA).

"Bioconjugation linker," as used herein refers to any chemical group that facilitates conjugation of an oligonucleotide synthesized and released from a covalently-modified, macroporous CPSG disclosed herein to any of a variety of biologically relevant compounds, such as lipids, nucleic acids, peptides, proteins and the like. To facilitate conjugation of an oligonucleotide synthesized and released from a covalently-modified, macroporous CPSG disclosed herein, the bioconjugation linker can be covalently attached to Y by N(H), S, O or a dithiolane or dioxalane of one of the following structural formulas: 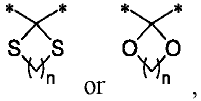 wherein n is 1, 2, 3 or 4 and * indicates the points of attachment of the dithiolane or dioxalane to O and Y. Examples of bioconjugation linkers include, but are not limited to, (C₁-C₂₅)alkylene, (C₁-C₂₅)alkenylene, (C₁-C₂₅)alkynylene, (C₁-C₂₅)heteroalkylene, (C₁-C₂₅)heteroalkenylene and (C₁-C₂₅)heteroalkynylene, covalently attached to Y by N(H), S, O or a dithiolane or dioxalane of one of the following structural formulas: 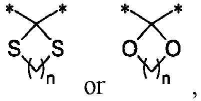 wherein n is 1, 2, 3 or 4 and * indicates the points of attachment of the dithiolane or dioxalane to O and Y.

In an aspect of any of the aforementioned aspects or embodiments, the macroporous CPSG has a mean particle size of from about 50 microns to about 500 microns, from about 70 microns to about 200 microns or from about 70 microns to about 150 microns.

In an aspect of any of the aforementioned aspects or embodiments, the macroporous CPSG has a mean pore size of greater than or about 500 Å, greater than or about 500 Å to about 5,000 Å, greater than or about 500 Å to about 1,000 Å, greater than or about 750 Å to about 1,000 Å, about 800 Å or about 1,000 Å.

In an aspect of any of the aforementioned aspects or embodiments, there are less than or about 100 micromoles of the moiety per gram of the covalently-modified, macroporous CPSG, from about 25 micromoles of the moiety per gram of the covalently-modified, macroporous CPSG to about 100 micromoles of the moiety per gram of the covalently-modified macroporous CPSG, from about 35 micromoles of the moiety per gram of the covalently-modified, macroporous CPSG to about 80 micromoles of the moiety per gram of the covalently-modified, macroporous CPSG or from about 40 micromoles of the moiety per gram of the covalently-modified, macroporous CPSG to about 75 micromoles per gram of the covalently-modified, macroporous CPSG.

In an aspect of any of the aforementioned aspects or embodiments, W is (R¹)₂Si-(C₁-C₂₅)alkylene-N(H)^ or (R¹)₂Si-(C₁-C₂₅)heteroalkylene-N(H)^. In a further aspect of any of the aforementioned aspects or embodiments, W is 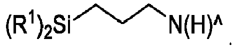 In an alternative further aspect of any of the aforementioned aspects or embodiments, W is 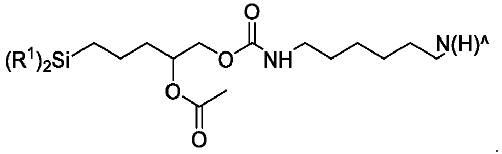 In yet another further aspect of any of the aforementioned aspects or embodiments, W is

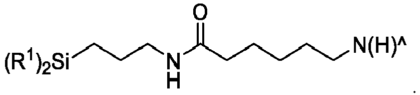

In an aspect of any of the aforementioned aspects or embodiments, Y is a linker selected from succinyl, oxalyl or hydroquinone-*O,O*'-diacetyl (*e.g*., succinyl).

In an aspect of any of the aforementioned aspects or embodiments, Z is N(H).

In an aspect of any of the aforementioned aspects or embodiments, R is H. In an alternative aspect of any of the aforementioned aspects or embodiments, R is 4,4'-dimethoxytrityl.

In an aspect of any of the aforementioned aspects or embodiments, the macroporous CPSG has a density of from about 0.35 g/mL to about 0.75 g/mL *(e.g.,* from about 0.4 g/mL to about 0.6 g/mL, or about 0.5 g/mL).

### Methods of Synthesizing Oligonucleotides Using Covalently-Modified, Macroporous Controlled Porosity Silica Gel

Covalently-modified, macroporous CPSG, such as any of the covalently-modified, macroporous CPSG disclosed herein, is suitable for the synthesis of oligonucleotides. Accordingly, one embodiment is a method for synthesizing an oligonucleotide, comprising providing a covalently-modified, macroporous CPSG disclosed herein (*e.g*., macroporous CPSG covalently modified with any of the moieties described herein, such as the moieties of structural formulas la, Ib, Ic, II and III); attaching one or more nucleotides to the macroporous CPSG, thereby synthesizing an oligonucleotide covalently attached to the macroporous CPSG; and cleaving the oligonucleotide covalently attached to the macroporous CPSG from the macroporous CPSG, thereby synthesizing the oligonucleotide. In some aspects of this embodiment, the method further comprises isolating and/or purifying the oligonucleotide.

Methods for attaching one or more nucleotides to a solid support, cleaving the oligonucleotide from the solid support and isolating and/or purifying the oligonucleotide are well-known in the art, and described, for example, in Richard T. Pon, Current Protocols in Nucleic Acid Chemistry (2000) 3.1.1-3.1.28, the entire content of which is incorporated herein by reference.

### Methods of Making Covalently-Modified, Macroporous Controlled Porosity Silica Gel

Another embodiment is a method of making covalently-modified, macroporous CPSG, such as any of the covalently-modified, macroporous CPSG disclosed herein. In one aspect, the method comprises covalently modifying one or more hydroxyl groups of macroporous CPSG with a spacer to obtain microporous CPSG covalently modified with a spacer; capping unreacted hydroxyl groups of the macroporous CPSG covalently modified with the spacer (*e.g*., with a silylating agent) to obtain capped and functionalized macroporous CPSG; and reacting capped and functionalized macroporous CPSG with a linker covalently attached to a nucleoside in the presence of a coupling reagent in an organic solvent *(e.g.,* a polar, aprotic organic solvent), thereby making covalently-modified, macroporous CPSG. A person of ordinary skill in the art will be able to employ appropriate protecting group strategies, as necessary.

In another aspect, the method comprises covalently modifying one or more hydroxyl groups of macroporous CPSG with a spacer selected from (R²)₃Si-(C₁-C₂₅)alkylene-Z¹, (R²)₃Si-(C₂-C₂₅)alkenylene-Z¹, (R²)₃Si-(C₂-C₂₅)alkynylene-Z¹, (R²)₃Si-(C₁-C₂₅)heteroalkylene-Z¹, (R²)₃Si-(C₂-C₂₅)heteroalkenylene-Z¹ or (R²)₃Si-(C₂-C₂₅)heteroalkynylene-Z¹ *(e.g.,* (R²)₃Si-(C₁-C₂₅)alkylene-Z¹ or (R²)₃Si-(C₁-C₂₅)heteroalkylene-Z¹), wherein each R² is independently -O(C₁-C₁₀)alkyl or -O(C₆-C₁₂)aryl; Z¹ is NH₂, OP¹ or SP² (*e.g*., NH₂); P¹ is a hydroxyl protecting group; and P² is a sulfhydryl protecting group, to obtain macroporous CPSG covalently modified with a spacer selected from 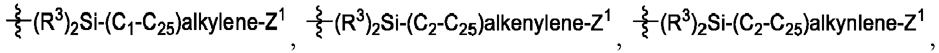 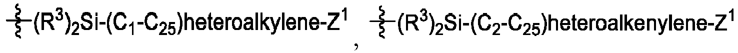 or 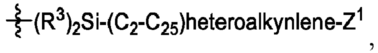 respectively, wherein 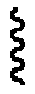 indicates the point of attachment of the spacer to the macroporous CPSG; each R³ is independently an additional point of attachment of the spacer to the macroporous CPSG, or R²; and R² and Z¹ are as described above. Unsilylated hydroxyl groups of the macroporous CPSG covalently modified with the spacer are capped (*e.g*., with a silylating agent), and P¹ and P², if present, are removed with a deprotecting agent to obtain capped and functionalized macroporous CPSG. The capped and functionalized macroporous CPSG is reacted with a linker covalently attached to a nucleoside in the presence of a coupling reagent in an organic solvent (*e.g*., a polar, aprotic organic solvent), thereby making covalently-modified, macroporous CPSG.

In yet another aspect, the method comprises covalently modifying one or more hydroxyl groups of macroporous CPSG with a spacer selected from (R²)₃Si-(C₁-C₂₅)alkylene-Z¹, (R²)₃Si-(C₂-C₂₅)alkenylene-Z¹, (R²)₃Si-(C₂-C₂₅)alkynylene-Z¹, (R²)₃Si-(C₁-C₂₅)heteroalkylene-Z¹, (R²)₃Si-(C₂-C₂₅)heteroalkenylene-Z¹ or (R²)₃Si-(C₂-C₂₅)heteroalkynylene-Z¹ *(e.g.,* (R²)₃Si-(C₁-C₂₅)alkylene-Z¹ or (R²)₃Si-(C₁-C₂₅)heteroalkylene-Z¹), wherein each R² is independently -O(C₁-C₁₀)alkyl or -O(C₆-C₁₂)aryl; Z¹ is NH₂, OP¹ or SP² (*e.g*., NH₂); P¹ is a hydroxyl protecting group; and P² is a sulfhydryl protecting group, to obtain macroporous CPSG covalently modified with a spacer selected from 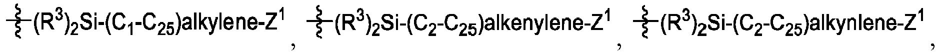 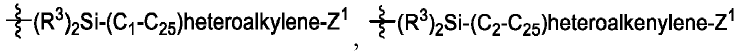 or 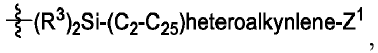 respectively, wherein 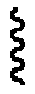 indicates the point of attachment of the spacer to the macroporous CPSG; each R³ is independently an additional point of attachment of the spacer to the macroporous CPSG, or R²; and R² and Z¹ are as described above. Unsilylated hydroxyl groups of the macroporous CPSG covalently modified with a spacer are capped (*e.g*., with a silylating agent), and P¹ and P², if present, are removed with a deprotecting agent to obtain capped and functionalized macroporous CPSG. The capped and functionalized macroporous CPSG is reacted with a compound of one of the following structural formulas: 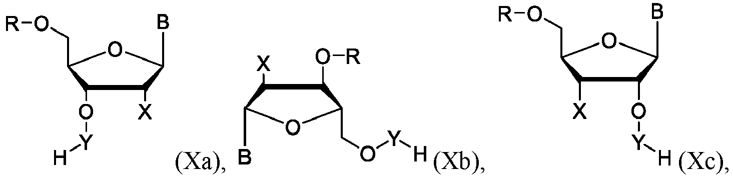 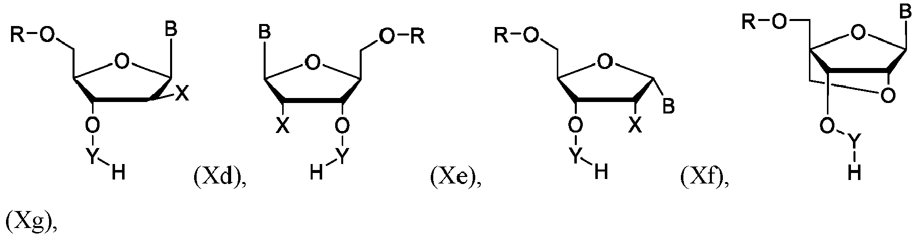 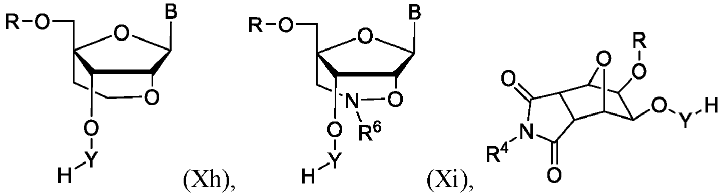 (XI) or R-O-Q-Y-H (XII), wherein R is 4,4'-dimethoxytrityl and R⁴, R⁶, B, Q, X and Y are as described in any embodiment or aspect described hereinabove, in the presence of a coupling reagent in an organic solvent *(e.g.,* a polar, aprotic organic solvent), thereby making covalently-modified, macroporous CPSG.

Coupling reagents mediate amide bond formations between carboxylic acids or their corresponding carboxylates and amines. Coupling reagents are well-known in the art. Examples include 2-(1*H*-benzotriazol-1-yl)-1,1,3,3-tetramethyluronium hexafluorophosphate (HBTU), 3-(ethyliminomethyleneamino)-*N*,*N*-dimethylpropan-1-amine (EDC), 1-[bis(dimethylamino)methylene]-1H-1,2,3-triazolo[4,5-b]pyridinium 3-oxide hexafluorophosphate (HATU), (Benzotriazol-1-yloxy)tris(dimethylamino)phosphonium hexafluorophosphate (BOP) and benzotriazol-1-yl-oxytripyrrolidinophosphonium hexafluorophosphate (PyBOP).

Oftentimes, coupling reagents are used in conjunction with an amine base, such as diisopropylethylamine, pyridine or triethylamine. Thus, in some embodiments, the capped and functionalized macroporous CPSG is reacted with a compound of one of structural formulas Xa, Xb, Xc, XI or XII in the presence of a coupling reagent and a base (*e.g*., an amine base) in an organic solvent (*e.g*., a polar, aprotic organic solvent).

Examples of organic solvents include nonpolar organic solvents (*e.g*., hexanes, cyclohexane, toluene, 1,4-dioxane), polar, aprotic organic solvents (*e.g*., dimethylsulfoxide, dimethylformamide, tetrahydrofuran, ethyl acetate, methylene chloride, acetone, acetonitrile), ethers (*e.g*., diethyl ether, methyl tert-butyl ether, 1,4-dioxane) and alcohols (*e.g*., methanol, ethanol, isopropanol).

For example, macroporous CPSG can be chemically modified by treating macroporous CPSG with 3-aminopropyl triethoxysilene, followed by capping the unreacted hydroxyl groups with a capping agent (*e.g*., a silylating agent such as trimethylsilyl chloride) in accordance with the procedure depicted in Scheme 1.

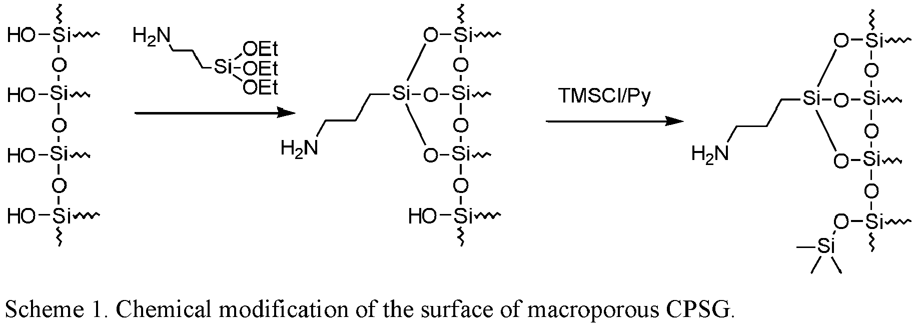

A first nucleoside can be preloaded onto the aminopropyl-modified, macroporous CPSG produced in Scheme 1 via its corresponding linker (*e.g*., succinate) in accordance with the procedure depicted in Scheme 2, wherein CPSG is controlled porosity silica gel *(e.g.,* macroporous CPSG) and DMT is 4,4'-dimethoxytrityl.

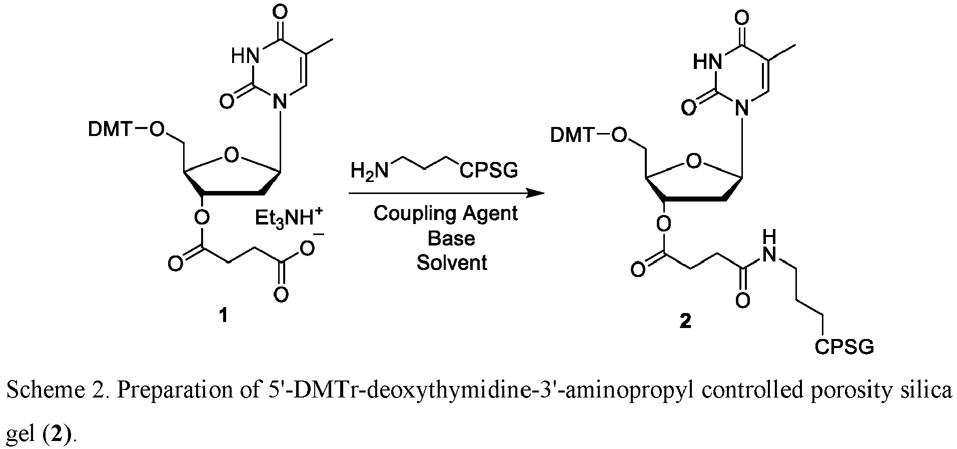

Alternatively, a universal linker that can be used for any type of oligonucleotide can be loaded onto the aminopropyl-modified, macroporous controlled porosity silica gel produced in Scheme 1 via its corresponding linker (*e.g*., succinate) in accordance with the procedure depicted in Scheme 3.

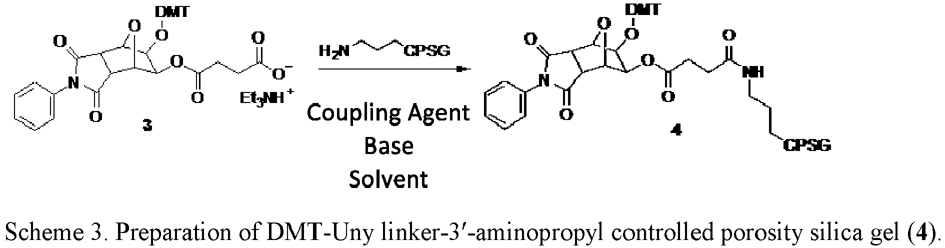

### NUMBERED EMBODIMENTS

1. Macroporous controlled porosity silica gel (CPSG) covalently modified with a moiety represented by one of the following structural formulas: 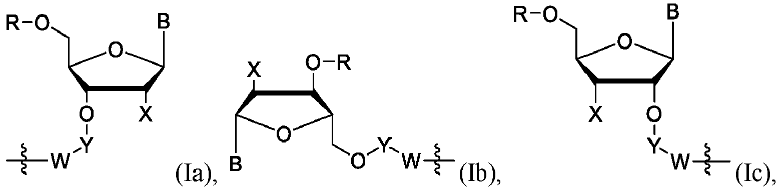 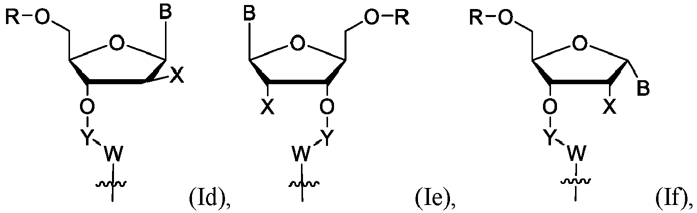 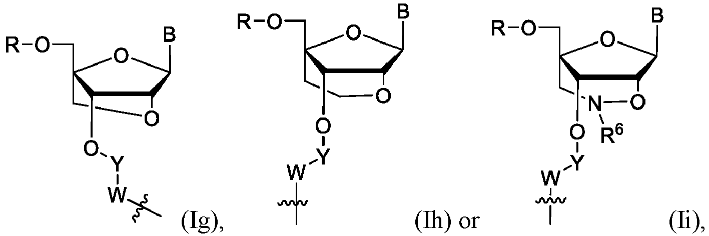 wherein:
   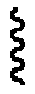 indicates the point of attachment of the moiety to macroporous CPSG;
   B is a nucleoside base radical selected from 9-(N⁶-benzoyladeninyl)-, 9-(N⁶-(N,N-dimethylformamidinyl)-adeninyl), 1-(N⁴-acetylcytosinyl)-, 1-(N⁴-benzoylcytosinyl)-, 1-(N⁴-isobutyrylcytosinyl)-,1-(N⁴-(N,N-dimethylformamidinyl)cytosinyl)-, 1-(N⁴-phenoxyacetylcytosinyl)-, 1-(N⁴*-tert-*butylphenoxyacetylcytosinyl)-, 1-(N⁴-isopropyl phenoxyacetylcytosinyl)-, 9-(N²-isobutyrylguaninyl)-,9-(N²-*tert*-butylphenoxyacetylguaninyl)-,9-(N²-isopropylphenoxyacetylguaninyl)-, 9-(N⁶-(N,N-dimethylformamidinyl)-guaninyl)-, 1-uracilyl- or pseudouracilyl; a modified nucleoside base radical selected 1-(N⁴-benzoyl-5-methylcytosinyl)-,1-(N⁴-(N,N-dimethylformamidinyl)-5-methylcytosinyl)-, 1-(N⁴-acetyl-5-methyl cytosinyl)-, 1 -(5-methyl-uracilyl)-, 1-(5-fluoro-uracilyl)-, 1-(N⁴-benzoyl-5-fluorocytosinyl)-, 9-(N⁶-benzoyl-7-deazaadeninyl)-, 9-(N⁶-(N,N-dimethylformamidinyl)-7-deazaadeninyl)-, 9-(N²-isobutyryl-7-deazaguaninyl)-, 9-(N²-(N,N-dimethylformamidinyl)-7-deazaguaninyl)-, 1-(N⁴-benzoyl-5-bromo-cytosinyl)-, 1-(5-bromo-uracilyl)-, 1-(5-iodo-uracilyl)-, 1-(5-vinyl-uracilyl)-, 1-(N³-methyl-uracilyl)-, 1-(N⁴-benzoyl-N³-methylcytosinyl)-, 1-(N³-methyl-5-methyluracilyl)-, 9-purinyl, 9-(N²-phenoxyacetyl-2-aminopurinyl)-, 9-(N^{2,}N⁶-diphenoxyacetyl-2,6-diaminopurinyl)-, 9-(N⁶-benzoyl-8-bromoadeninyl)-, 9-(N⁶-benzoyl-8-oxoadeninyl)-, 9-(N¹-methyl-N⁶-(9-fluorenylmethyloxycarbonyl)-7-deazaadeninyl)-, 9-(N²-isobutyryl-8-oxoguaninyl)-, 9-(N⁶-(N,N-dimethylformamidinyl)-8-oxoguaninyl)-, 9-(etheno-adeninyl)-, 1-(etheno-cytosinyl)-, 9-(hypoxanthinyl)-, 9-(8-bromohypoxanthinyl)-, 9-(N²-methylhypoxanthinyl)-, 5-(1,2-diacetyloxyethyl)-uracilyl, *N*³-acetyl-5-(1,2-diacetyloxyethyl)-cytosinyl, 5-acetoxymethyluracilyl or *N*³-acetyl-5-cyanoethoxymethyl-cytosinyl; or H;
   R is H or 4,4'-dimethoxytrityl;
   W is a spacer selected from (R¹)₂Si-(C₁-C₂₅)alkylene-Z^, (R¹)₂Si-(C₂-C₂₅)alkenylene-Z^, (R¹)₂Si-(C₂-C₂₅)alkynylene-Z^, (R¹)₂Si-(C₁-C₂₅)heteroalkylene-Z^, (R¹)₂Si-(C₂-C₂₅)heteroalkenylene-Z^ or (R¹)₂Si-(C₂-C₂₅)heteroalkynylene-Z^;
   ^ indicates the point of attachment of W to Y;
   each R¹ is independently an additional point of attachment of W to the macroporous CPSG, or -O(C₁-C₁₀)alkyl or -O(C₆-C₁₂)aryl;
   R⁶ is methyl or acetyl;
   X is hydrogen, halogen, hydroxyl, OP, thio, (C₁-C₁₀)alkoxy, (C₁-C₁₀)thioalkoxy, NH₂, N(H)P³ or NP³₂;
   P is -OCH₃, -OCH₂CH₃, -OCH₂CH₂OCH₃, -OCH₂CH=CH₂ or -OCH₂C≡CH, or a hydroxyl protecting group;
   each P³ is independently an amino protecting group or two P³, taken together with the nitrogen to which they are attached, form a cyclic di-protected amino;
   Y is a linker selected from succinyl, oxalyl, malonyl, diglycolyl or hydroquinone-*O*,*O'*-diacetyl; and
   Z is N(H), O or S.
2. Macroporous controlled porosity silica gel (CPSG) covalently modified with a moiety represented by the following structural formula: 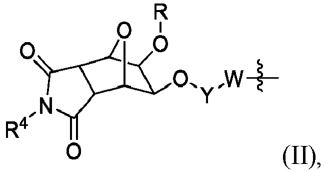 wherein:
   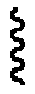 indicates the point of attachment of the moiety to macroporous CPSG;
   R is H or 4,4'-dimethoxytrityl;
   W is a spacer selected from (R¹)₂Si-(C₁-C₂₅)alkylene-Z^, (R¹)₂Si-(C₂-C₂₅)alkenylene-Z^, (R¹)₂Si-(C₂-C₂₅ )alkynylene-Z^, (R¹)₂Si-(C₁-C₂₅)heteroalkylene-Z^, (R¹)₂Si-(C₂-C₂₅ )heteroalkenylene-Z^ or (R¹)₂Si-(C₂-C₂₅)heteroalkynylene-Z^;
   ^ indicates the point of attachment of W to Y;
   each R¹ is independently an additional point of attachment of W to the macroporous CPSG, or -O(C₁-C₁₀)alkyl or -O(C₆-C₁₂)aryl;
   R⁴ is (C₁-C₂₅)alkyl, or phenyl optionally substituted with one or more R⁵;
   R⁵, for each occurrence, is independently halo, (C₁-C₅)alkyl, (C₁-C₅)haloalkyl, (Ci-C₅)alkoxy or (C₁-C₅)haloalkoxy;
   Y is a linker selected from succinyl, oxalyl, malonyl, diglycolyl or hydroquinone-*O*,*O'*-diacetyl; and
   Z is N(H), O or S.
3. Macroporous controlled porosity silica gel (CPSG) covalently modified with one or more moieties, each independently represented by the following structural formula: 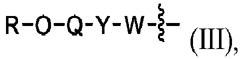 wherein:
   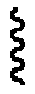 indicates the point of attachment of each moiety to macroporous CPSG;
   Q comprises a chromophore selected from fluorescein, carboxytetramethylrhodamine (TAMRA), rhodamine X (ROX), sulforhodamine 101 acid chloride (Texas Red), Cy3, Cy5, dabcyl, iQ2™ or iQ4™; a ligand selected from cholesterol, tocopherol, palmitic acid, biotin or psoralen; or a bioconjugation linker covalently attached to Y by N(H), S, O or a dithiolane or dioxalane of one of the following structural formulas: 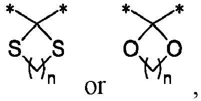 wherein n is 1, 2, 3 or 4 and * indicates the points of attachment of the dithiolane or dioxalane to O and Y;
   R is H or 4,4'-dimethoxytrityl;
   W is a spacer selected from (R¹)₂Si-(C₁-C₂₅)alkylene-Z^, (R¹)₂Si-(C₂-C₂₅)alkenylene-Z^, (R¹)₂Si-(C₂-C₂₅ )alkynylene-Z^, (R¹)₂Si-(C₁-C₂₅)heteroalkylene-Z^, (R¹)₂Si-(C₂-C₂₅ )heteroalkenylene-Z^ or (R¹)₂Si-(C₂-C₂₅)heteroalkynylene-Z^;
   ^ indicates the point of attachment of W to Y;
   each R¹ is independently an additional point of attachment of W to the macroporous CPSG, or -O(C₁-C₁₀)alkyl or -O(C₆-C₁₂)aryl;
   Y is a linker selected from succinyl, oxalyl, malonyl, diglycolyl or hydroquinone-*O*,*O'*-diacetyl; and
   Z is N(H), O or S.
4. The macroporous CPSG of any one of embodiments 1-3, wherein the macroporous CPSG has a mean particle size of from about 50 microns to about 500 microns.
5. The macroporous CPSG of embodiment 4, wherein the macroporous CPSG has a mean particle size of from about 70 microns to about 200 microns.
6. The macroporous CPSG of any one of embodiments 1-5, wherein the macroporous CPSG has a mean pore size of greater than or about 500 Å to about 5,000 Å.
7. The macroporous CPSG of embodiment 6, wherein the macroporous CPSG has a mean pore size of greater than or about 500 Å to about 1,000 Å.
8. The macroporous CPSG of embodiment 7, wherein the macroporous CPSG has a mean pore size of greater than or about 750 Å to about 1,000 Å.
9. The macroporous CPSG of any one of embodiments 1-8, wherein there are less than or about 100 micromoles of the moiety per gram of the covalently-modified, macroporous CPSG.
10. The macroporous CPSG of embodiment 9, wherein there are from about 25 micromoles of the moiety per gram of the covalently-modified, macroporous CPSG to about 100 micromoles of the moiety per gram of the covalently-modified, macroporous CPSG.
11. The macroporous CPSG of embodiment 10, wherein there are from about 35 micromoles of the moiety per gram of the covalently-modified, macroporous CPSG to about 80 micromoles of the moiety per gram of the covalently-modified, macroporous CPSG.
12. The macroporous CPSG of any one of embodiments 1-11, wherein W is (R¹)₂Si-(C₁-C₂₅)alkylene-N(H)^ or (R¹)₂Si-(C₁-C₂₅)heteroalkylene-N(H)^.
13. The macroporous CPSG of embodiment 12, wherein W is 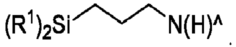
14. The macroporous CPSG of embodiment 12, wherein W is 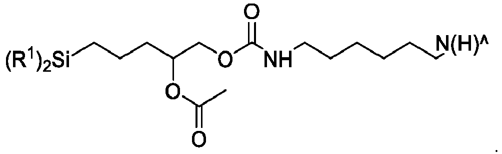
15. The macroporous CPSG of any one of embodiments 1-14, wherein Y is a linker selected from succinyl, oxalyl or hydroquinone-*O,O*'-diacetyl.
16. The macroporous CPSG of embodiment 15, wherein Y is succinyl.
17. The macroporous CPSG of any one of embodiments 1-16, wherein Z is N(H).
18. The macroporous CPSG of any one of embodiments 1-17, wherein the macroporous CPSG has a density of from about 0.35 g/mL to about 0.75 g/mL.
19. A method of synthesizing an oligonucleotide, comprising:
   providing macroporous CPSG of any one of embodiments 1-18;
   attaching one or more nucleotides to the macroporous CPSG, thereby synthesizing an oligonucleotide covalently attached to the macroporous CPSG; and
   cleaving the oligonucleotide covalently attached to the macroporous CPSG from the macroporous CPSG,
   thereby synthesizing the oligonucleotide.
20. A method of making the macroporous CPSG of any one of embodiments 1-18, comprising:
   covalently modifying one or more hydroxyl groups of macroporous CPSG with a spacer selected from (R²)₃Si-(C₁-C₂₅)alkylene-Z¹, (R²)₃Si-(C₁-C₂₅)alkenylene-Z¹, (R²)₃Si-(C₁-C₂₅)alkynylene-Z¹, (R²)₃Si-(C₁-C₂₅)heteroalkylene-Z¹, (R²)₃Si-(C₁-C₂₅)heteroalkenylene-Z¹ or (R²)₃Si-(C₁-C₂₅)heteroalkynylene-Z¹, wherein each R² is independently -O(C₁-C₁₀)alkyl or -O(C₆-C₁₂)aryl; Z¹ is NH₂, OP¹ or SP²; P¹ is a hydroxyl protecting group; and P² is a sulfhydryl protecting group, to obtain macroporous CPSG covalently modified with a spacer selected from 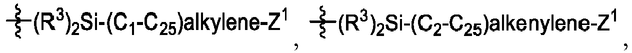 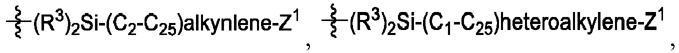 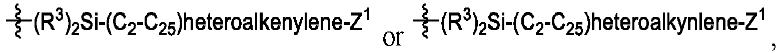 respectively, wherein 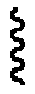 indicates the point of attachment of the spacer to the macroporous CPSG; each R³ is independently an additional point of attachment of the spacer to the macroporous CPSG, or R²; and R² and Z¹ are as described above;
   capping unsilylated hydroxyl groups of the macroporous CPSG covalently modified with a spacer with a silylating agent, and removing P¹ and P², if present, with a deprotecting agent, to obtain capped and functionalized macroporous CPSG; and
   reacting a compound of one of the following structural formulas: 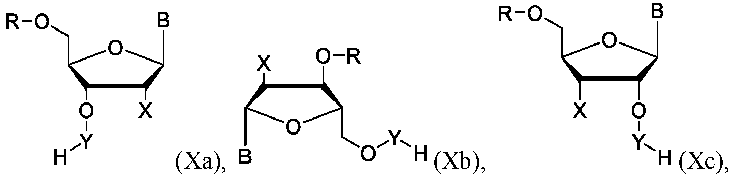 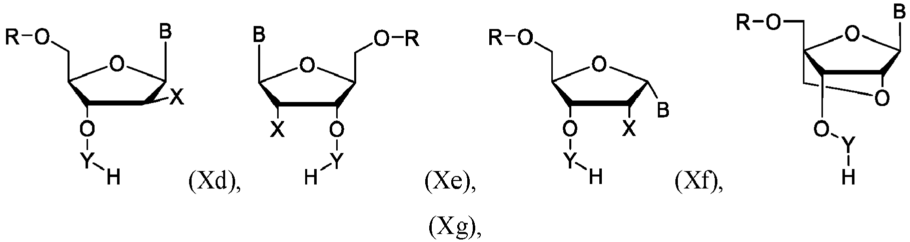 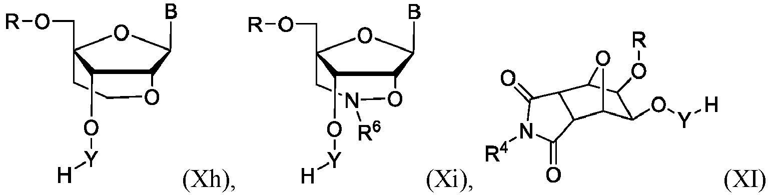 or

      R-O-Q-Y-H (XII),
   wherein R is 4,4'-dimethoxytrityl and R⁴, R⁶, B, Q, X and Y are as described above,
   with the capped and functionalized macroporous CPSG in the presence of a coupling reagent in an organic solvent,
   thereby making the macroporous CPSG of any one of embodiments 1-18.

### EXEMPLIFICATION

### Example 1. Preparation of 5'-DMT-deoxythymidine 3'-aminopropyl silica gel (2).

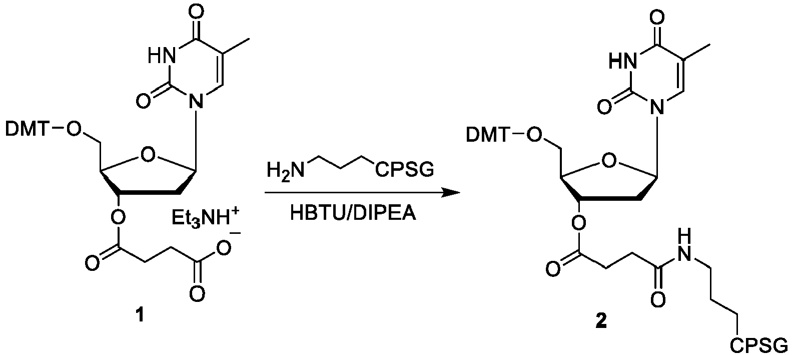

A solution of 5'-O-DMT-3'-succinyldeoxythymidine (1, 0.43 g, 0.575 mmoles) in 20 mL of dry acetonitrile was added to 5 g of aminopropyl modified silica gel. To the reaction slurry that was placed on a rotary shaker was added diisopropylethylamine (DIPEA; 0.41 ml, 2.4 mmoles) and (2-(1*H*-benzotriazol-1-yl)-1,1,3,3-tetramethyluronium hexafluorophosphate (HBTU; 0.24 g, 0.63 mmoles). After 12 hours, the supernatant solution was removed and the silica gel was washed with acetonitrile (3x50 ml).

25 ml of a solution of Cap A (Acetic anhydride/Pyridine/THF, 1:1:8) and 25 ml of pyridine was added to the washed silica gel. After 12 hours, the supernatant solution was again removed, and the solid support was washed with acetonitrile (3x50 ml) and diethyl ether (2x50 ml). The resulting solid support was dried under vacuum for 12 hours. The nucleoside loading was estimated by DMT removal procedure to be 75 µmole/g.

### Example 2. Preparation of DMT-Uny linker silica gel (4)

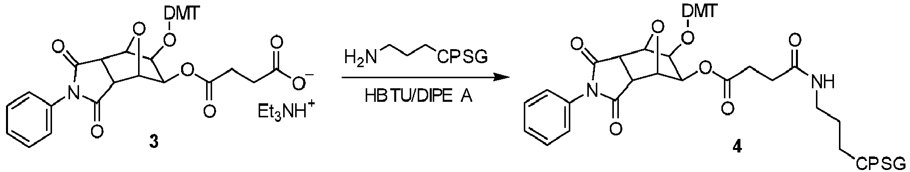

A solution of DMT-Uny linker succinate (3, 1.30 g, 1.6 mmoles) in 50 mL of dry acetonitrile was added to 10 g of aminopropyl modified silica gel. To the reaction slurry that was placed on rotary shaker was added diisopropylethylamine (0.41 ml, 2.4 mmoles) and HBTU (0.24 g, 0.63 mmoles). After 12 hours, the supernatant solution was removed and the silica gel was washed with acetonitrile (3x50 ml).

25 ml of a solution of Cap A and 25 ml of pyridine was added to the washed silica gel. After 12 hours, the supernatant solution was again removed, and the solid support was washed with acetonitrile (3x50 ml) and diethyl ether (2x50 ml). The resulting solid support was dried under vacuum for 12 hours. The nucleoside loading was estimated by DMTr removal procedure to be 60 µmole/g.

### Example 3. Oligonucleotide syntheses using covalently-modified macroporous controlled porosity silica gel

The solid supports from Examples 1 and 2, as well as an additional solid support, rU, were used to synthesize oligonucleotides corresponding to SEQ ID NOS:1-3 at 1 µmole scale using an ABI 8900 synthesizer, or at 200 µmole scale using an Akta 100 Plus synthesizer and standard DNA or RNA synthesis protocols. The sequences of SEQ ID NOS:1-3 are set forth in Table 1.

**Table 1.**

| SEQ ID NO | Oligonucleotide Sequence |
|---|---|
| 1 | TCAGTGCACGATAGTCGCATT |
| 2 | CACTCCACTACAACTACATGTGTAACAGTTCCTCCATATGTTCCTCCTCCAT CCACTACTTGCCATCCACTACAACTACATGTGTAACAGTTCCTCCATA |
| 3 | rGrArGrUrUrCrArUrArGrCrU |

The oligonucleotides were processed using standard conditions, and the yield and quality of the crude oligonucleotides were analyzed by ion-exchange high-performance liquid chromoatography (IE-HPLC) and electrospray ionization mass spectroscopy (ESIMS). The results of the analyses are summarized in Table 2, wherein FLP is full-length product and rU corresponds to macroporous controlled porosity silica gel (2) wherein the 5'-O-DMT-deoxythymidine is replaced by 5'-O-DMT-2'-O-*tert*-butyldimethylsilyluridine.

**Table 2.**

| | SEQ ID NO: 1 | SEQ ID NO:2 | SEQ ID NO: 1 | SEQ ID NO:3 |
|---|---|---|---|---|
| | Silica gel: **(1)** | Silica gel : **(1)** | Silica gel: **(3)** | Silica gel: rU |
| | Pore size: 800 Å | Pore size: 1,000 Å | Pore size: 800 Å | Pore size: 800 Å |
| | Loading: 75 | Loading: 40 | Loading: 60 | Loading: 46 |
| | µmole/g | µmole/g | µmole/g | µmole/g |
| Synthesis scale | 225 µmole | 1 µmole | 1 µmole | 160 µmole |
| FLP | 85% | 30.4% | 91.2% | 88.8% |
| Recovery | 155 µmoles | 275 nmoles | 650 nmoles | 88 µmoles |
| N-1 | 0.7% | - | 0.3% | 0.9% |
| N+1 | 0.9% | - | 0.3% | 0.8% |
| Coupling efficiency | 99.2% | 98.8% | 99.5% | 99.0% |

### Example 4. Density characteristics of macroporous CPSG

1.5-Grams of aminopropyl-modified CPG (1,000 Å; d:0.25g/mL) occupied a height of 46 mm in a 3-mL oligonucleotide synthesis column, thereby filling the space in the oligonucleotide synthesis column almost completely. 1.5-Grams of aminopropyl-modified polystyrene-based solid support (700 Å; Nitto phase; d:0.18g/mL) for oligonucleotide synthesis similarly occupied almost all of the space in the 3-mL oligonucleotide synthesis column, reaching a height of 45 mm. In contrast, 1.5-grams of aminopropyl-modified, macroporous CPSG (1,000 Å; d:0.5g/mL) occupied only 29 mm of column height in the 3-mL oligonucleotide synthesis column. Thus, much more macroporous CPSG *(e.g.,* covalently-labeled, macroporous CPSG) can be filled in a given oligonucleotide synthesis column, and a larger scale oligonucleotide synthesis achieved per column, resulting in greater yield of synthesized oligonucleotide.

### REFERENCES

Flora Chow, Tomas Kempet and Gunnar Palm, Nucleic Acid Res, 1981, 9, 2807-2817.
V.Kohli, A.Balland, M.Wintzerith, R.Sauerwald, A. Staub and J.P.Lecocq, Nucleic Acid Res, 1981, 10, 7439-7448.
Richard T. Pon, Current Protocols in Nucleic Acid Chemistry, 2000, unit 3.1.1.
G.Bruno F.Gasparrini D.Misiti E.Arrigoni-Martelli M.Bronzetti, Journal of Chromatography A, 1990, 504, 319-333.
Laikhter, A., Srivastava, S., and Srivastava, U.S. Patent 7,956,169.
Laikhter, A. 2015. Overview of qPCR molecular probes. Curr. Protoc. Essential Lab. Tech. 11:10.5.1-10.5.10.

The teachings of all patents, published applications and references cited herein are incorporated by reference in their entirety.

While example embodiments have been particularly shown and described, it will be understood by those skilled in the art that various changes in form and details may be made therein without departing from the scope of the embodiments encompassed by the appended claims.

## Claims

1. Macroporous controlled porosity silica gel (CPSG) covalently modified with a moiety represented by one of the following structural formulas: 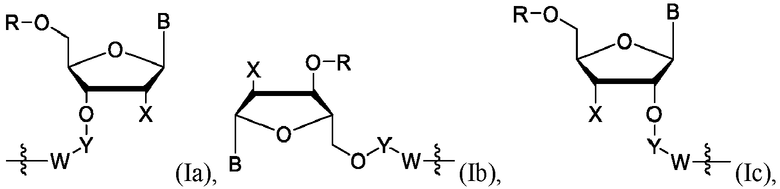 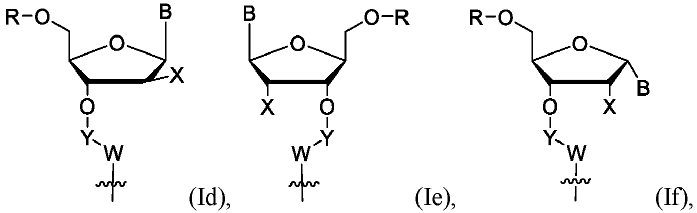 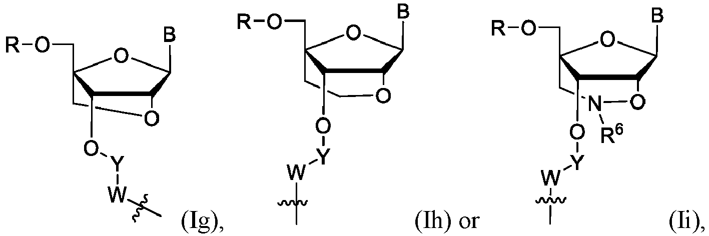 wherein:
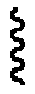 indicates the point of attachment of the moiety to macroporous CPSG;
B is a nucleoside base radical selected from 9-(N⁶-benzoyladeninyl)-, 9-(N⁶-(N,N-dimethylformamidinyl)-adeninyl), 1-(N⁴-acetylcytosinyl)-, 1-(N⁴-benzoylcytosinyl)-, 1-(N⁴-isobutyrylcytosinyl)-,1-(N⁴-(N,N-dimethylformamidinyl)cytosinyl)-, 1-(N⁴-phenoxyacetylcytosinyl)-, 1-(N⁴*-tert-*butylphenoxyacetylcytosinyl)-, 1-(N⁴-isopropyl phenoxyacetylcytosinyl)-, 9-(N²-isobutyrylguaninyl)-,9-(N²-*tert*-butylphenoxyacetylguaninyl)-,9-(N²-isopropylphenoxyacetylguaninyl)-, 9-(N⁶-(N,N-dimethylformamidinyl)-guaninyl)-, 1-uracilyl- or pseudouracilyl; a modified nucleoside base radical selected from 1-(N⁴-benzoyl-5-methylcytosinyl)-, 1-(N⁴-(N,N-dimethylformamidinyl)-5-methylcytosinyl)-, 1-(N⁴-acetyl-5-methylcytosinyl)-, 1-(5-methyl-uracilyl)-, 1-(5-fluoro-uracilyl)-, 1-(N⁴-benzoyl-5-fluorocytosinyl)-, 9-(N⁶-benzoyl-7-deazaadeninyl)-, 9-(N⁶-(N,N-dimethylformamidinyl)-7-deazaadeninyl)-, 9-(N²-isobutyryl-7-deazaguaninyl)-, 9-(N²-(N,N-dimethylformamidinyl)-7-deazaguaninyl)-, 1-(N⁴-benzoyl-5-bromo-cytosinyl)-, 1-(5-bromo-uracilyl)-, 1-(5-iodo-uracilyl)-, 1-(5-vinyl-uracilyl)-, 1-(N³-methyl-uracilyl)-, 1-(N⁴-benzoyl-N³-methylcytosinyl)-, 1-(N³-methyl-5-methyluracilyl)-, 9-purinyl, 9-(N²-phenoxyacetyl-2-aminopurinyl)-, 9-(N^{2,}N⁶-diphenoxyacetyl-2,6-diaminopurinyl)-, 9-(N⁶-benzoyl-8-bromoadeninyl)-, 9-(N⁶ -benzoyl-8-oxoadeninyl)-, 9-(N¹-methyl-N⁶-(9-fluorenylmethyloxycarbonyl)-7-deazaadeninyl)-, 9-(N²-isobutyryl-8-oxoguaninyl)-, 9-(N⁶-(N,N-dimethylformamidinyl)-8-oxoguaninyl)-, 9-(etheno-adeninyl)-, 1-(etheno-cytosinyl)-, 9-(hypoxanthinyl)-, 9-(8-bromohypoxanthinyl)-, 9-(N²-methylhypoxanthinyl)-, 5-(1,2-diacetyloxyethyl)-uracilyl, *N*³-acetyl-5-(1,2-diacetyloxyethyl)-cytosinyl, 5-acetoxymethyluracilyl or *N*³-acetyl-5-cyanoethoxymethyl-cytosinyl; or H;
R is H or 4,4'-dimethoxytrityl;
W is a spacer selected from (R¹)₂Si-(C₁-C₂₅)alkylene-Z^, (R¹)₂Si-(C₂-C₂₅)alkenylene-Z^, (R¹)₂Si-(C₂-C₂₅ )alkynylene-Z^, (R¹)₂Si-(C₁-C₂₅)heteroalkylene-Z^, (R¹)₂Si-(C₂-C₂₅ )heteroalkenylene-Z^ or (R¹)₂Si-(C₂-C₂₅)heteroalkynylene-Z^;
^ indicates the point of attachment of W to Y;
each R¹ is independently an additional point of attachment of W to the macroporous CPSG, or -O(C₁-C₁₀)alkyl or -O(C₆-C₁₂)aryl;
R⁶ is methyl or acetyl;
X is hydrogen, halogen, hydroxyl, OP, thio, (C₁-C₁₀)alkoxy, (C₁-C₁₀)thioalkoxy, NH₂, N(H)P³ or NP³₂;
P is -OCH₃, -OCH₂CH₃, -OCH₂CH₂OCH₃, -OCH₂CH=CH₂ or -OCH₂C≡CH, or a hydroxyl protecting group;
each P³ is independently an amino protecting group or two P³, taken together with the nitrogen to which they are attached, form a cyclic di-protected amino;
Y is a linker selected from succinyl, oxalyl, malonyl, diglycolyl or hydroquinone-*O*,*O'*-diacetyl; and
Z is N(H), O or S.

2. Macroporous controlled porosity silica gel (CPSG) covalently modified with a moiety represented by the following structural formula: 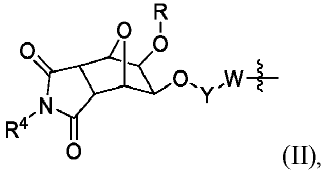 wherein:
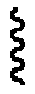 indicates the point of attachment of the moiety to macroporous CPSG;
R is H or 4,4'-dimethoxytrityl;
W is a spacer selected from (R¹)₂Si-(C₁-C₂₅)alkylene-Z^, (R¹)₂Si-(C₂-C₂₅)alkenylene-Z^, (R¹)₂Si-(C₂-C₂₅ )alkynylene-Z^, (R¹)₂Si-(C₁-C₂₅)heteroalkylene-Z^, (R¹)₂Si-(C₂-C₂₅ )heteroalkenylene-Z^ or (R¹)₂Si-(C₂-C₂₅)heteroalkynylene-Z^;
^ indicates the point of attachment of W to Y;
each R¹ is independently an additional point of attachment of W to the macroporous CPSG, or -O(C₁-C₁₀)alkyl or -O(C₆-C₁₂)aryl;
R⁴ is (C₁-C₂₅)alkyl, or phenyl optionally substituted with one or more R⁵;
R⁵, for each occurrence, is independently halo, (C₁-C₅)alkyl, (C₁-C₅)haloalkyl, (Ci-C₅)alkoxy or (C₁-C₅)haloalkoxy;
Y is a linker selected from succinyl, oxalyl, malonyl, diglycolyl or hydroquinone-*O*,*O'*-diacetyl; and
Z is N(H), O or S.

3. Macroporous controlled porosity silica gel (CPSG) covalently modified with a moiety represented by the following structural formula: 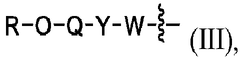 wherein:
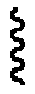 indicates the point of attachment of each moiety to macroporous CPSG;
Q comprises a chromophore selected from fluorescein, carboxytetramethylrhodamine (TAMRA), rhodamine X (ROX), sulforhodamine 101 acid chloride (Texas Red), Cy3, Cy5, dabcyl, IQ2 or IQ4; a ligand selected from cholesterol, tocopherol, palmitic acid, biotin or psoralen; or a bioconjugation linker covalently attached to Y by N(H), S, O or a dithiolane or dioxalane of one of the following structural formulas: 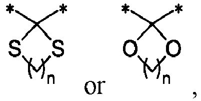 wherein n is 1, 2, 3 or 4 and * indicates the points of attachment of the dithiolane or dioxalane to O and Y;
R is H or 4,4'-dimethoxytrityl;
W is a spacer selected from (R¹)₂Si-(C₁-C₂₅)alkylene-Z^, (R¹)₂Si-(C₂-C₂₅)alkenylene-Z^, (R¹)₂Si-(C₂-C₂₅ )alkynylene-Z^, (R¹)₂Si-(C₁-C₂₅)heteroalkylene-Z^, (R¹)₂Si-(C₂-C₂₅ )heteroalkenylene-Z^ or (R¹)₂Si-(C₂-C₂₅)heteroalkynylene-Z^;
^ indicates the point of attachment of W to Y;
each R¹ is independently an additional point of attachment of W to the macroporous CPSG, or -O(C₁-C₁₀)alkyl or -O(C₆-C₁₂)aryl;
Y is a linker selected from succinyl, oxalyl, malonyl, diglycolyl or hydroquinone-*O*,*O'*-diacetyl; and
Z is N(H), O or S.

4. The macroporous CPSG of any one of claims 1-3, wherein the macroporous CPSG has a mean particle size of from about 50 microns to about 500 microns, preferably, of from about 70 microns to about 200 microns.

5. The macroporous CPSG of any one of claims 1-4, wherein the macroporous CPSG has a mean pore size of greater than or about 500 Å to about 5,000 Å, preferably, greater than or about 500 Å to about 1,000 Å, more preferably, greater than or about 750 Å to about 1,000 Å.

6. The macroporous CPSG of any one of claims 1-5, wherein there are less than or about 100 micromoles of the moiety per gram of the covalently-modified, macroporous CPSG, preferably, from about 25 micromoles of the moiety per gram of the covalently-modified, macroporous CPSG to about 100 micromoles of the moiety per gram of the covalently-modified, macroporous CPSG, more preferably, from about 35 micromoles of the moiety per gram of the covalently-modified, macroporous CPSG to about 80 micromoles of the moiety per gram of the covalently-modified, macroporous CPSG.

7. The macroporous CPSG of any one of claims 1-6, wherein W is (R¹)₂Si-(C₁-C₂₅)alkylene-N(H)^ or (R¹)₂Si-(C₁-C₂₅)heteroalkylene-N(H)^.

8. The macroporous CPSG of claim 7, wherein W is

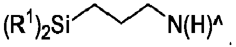

9. The macroporous CPSG of claim 7, wherein W is

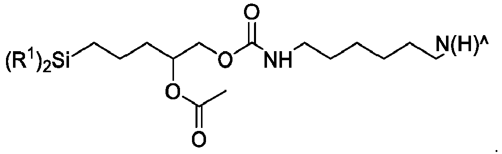

10. The macroporous CPSG of any one of claims 1-9, wherein Y is a linker selected from succinyl, oxalyl or hydroquinone-*O*,*O'*-diacetyl.

11. The macroporous CPSG of claim 10, wherein Y is succinyl.

12. The macroporous CPSG of any one of claims 1-11, wherein Z is N(H).

13. The macroporous CPSG of any one of claims 1-12, wherein the macroporous CPSG has a density of from about 0.35 g/mL to about 0.75 g/mL.

14. A method of synthesizing an oligonucleotide, comprising:
providing macroporous CPSG of any one of claims 1-13;
attaching one or more nucleotides to the macroporous CPSG, thereby synthesizing an oligonucleotide covalently attached to the macroporous CPSG; and
cleaving the oligonucleotide covalently attached to the macroporous CPSG from the macroporous CPSG,
thereby synthesizing the oligonucleotide.

15. A method of making the macroporous CPSG of any one of claims 1-13, comprising:
covalently modifying one or more hydroxyl groups of macroporous CPSG with a spacer selected from (R²)₃Si-(C₁-C₂₅)alkylene-Z¹, (R²)₃Si-(C₁-C₂₅)alkenylene-Z¹, (R²)₃Si-(C₁-C₂₅)alkynylene-Z¹, (R²)₃Si-(C₁-C₂₅)heteroalkylene-Z¹, (R²)₃Si-(C₁-C₂₅)heteroalkenylene-Z¹ or (R²)₃Si-(C₁-C₂₅)heteroalkynylene-Z¹, wherein each R² is independently -O(C₁-C₁₀)alkyl or -O(C₆-C₁₂)aryl; Z¹ is NH₂, OP¹ or SP²; P¹ is a hydroxyl protecting group; and P² is a sulfhydryl protecting group, to obtain macroporous CPSG covalently modified with a spacer selected from 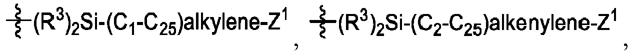 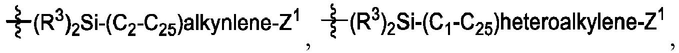 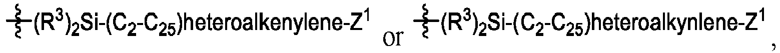 respectively, wherein 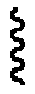 indicates the point of attachment of the spacer to the macroporous CPSG; each R³ is independently an additional point of attachment of the spacer to the macroporous CPSG, or R²; and R² and Z¹ are as described above;
capping unsilylated hydroxyl groups of the macroporous CPSG covalently modified with the spacer with a silylating agent, and removing P¹ and P², if present, with a deprotecting agent, to obtain capped and functionalized macroporous CPSG; and
reacting a compound of one of the following structural formulas: 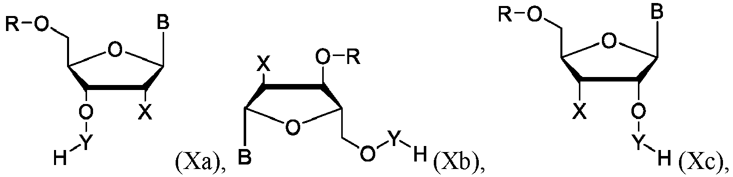 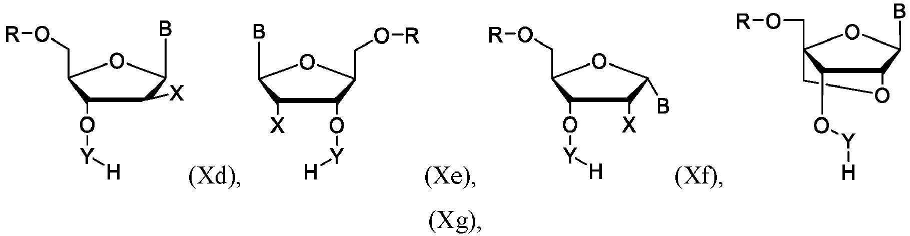 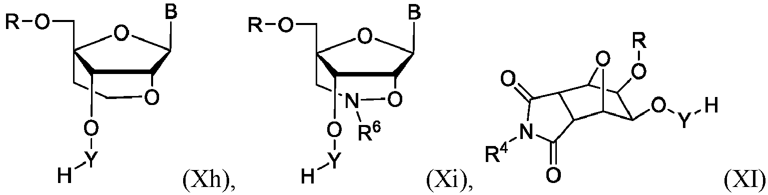 or
R-O-Q-Y-H (XII),
wherein R is 4,4'-dimethoxytrityl and R⁴, R⁶, B, Q, X and Y are as described in any of claims 1-3, 10 and 11,
with the capped and functionalized macroporous CPSG in the presence of a coupling reagent in an organic solvent,
thereby making the macroporous CPSG of any one of claims 1-13.
